# EUROPÄISCHE PATENTSCHRIFT

(11) **EP 2 742 962 B1**
(45) Veröffentlichungstag und Bekanntmachung des Hinweises auf die Patenterteilung: **25.12.2019**
(21) Anmeldenummer: 13178676.6
(22) Anmeldetag: 31.07.2013
(51) Int. Cl.: A61M 5/315, A61M 5/20

(54) **Autoinjektor mit einer Signalisierungseinrichtung am Ende der Produktausschüttung**
Autoinjector with a signalling device at the end of the product dispensing
Auto-injecteur doté d'un dispositif de signalisation à la fin de la distribution de produit

(30) Priorität: 22.03.2013 EP 13160614
(43) Veröffentlichungstag der Anmeldung: 18.06.2014
(73) Patentinhaber: TecPharma Licensing AG, 3401 Burgdorf (CH)
(72) Erfinder: Tschirren, Markus, 3400 Burgdorf (CH); Hirschel, Jürg, 3007 Bern (CH); Moser, Ulrich, 3412 Heimiswil (CH); Streit, Ursina, 3322 Schönbühl (CH)

(56) Entgegenhaltungen:
- EP-A1- 2 489 380
- WO-A1-2011/043714

## Beschreibung

Die Erfindung betrifft einen Autoinjektor, der oftmals auch als Autoinjektionsvorrichtung bezeichnet wird, mit dem ein in einem Produktbehälter enthaltenes Produkt nach dem Auslösen automatisch ausschüttbar ist. Bei dem flüssigen Produkt handelt es sich insbesondere um ein Medikament. Im Besonderen betrifft die Erfindung eine Signalisierungseinrichtung, die am Ende der Produktausschüttung ein akustisches oder taktiles Signal abgibt, um den Anwender über das Ende der erfolgten Produktausschüttung zu informieren.

Autoinjektoren sind im Stand der Technik bekannt. Ebenfalls sind Einrichtungen bekannt, die das Ende der Produktausschüttung signalisieren. Aus der DE 102008037310 A1 und der WO 2010/017650 A1 ist eine Injektionsvorrichtung bekannt, welche an der Kolbenstange ein Federelement aufweist, welches am Ende der automatischen Ausschüttung radial über Rippen am Gehäuse schnappt und den sogenannten "Endklick" bewirkt.

Weiter sind aus der DE 10359694 A1 und der WO 2004/047892 A1 Injektionsvorrichtungen, insbesondere Autoinjektoren bekannt, die eine Nadelschutzhülse aufweisen, die sich an einer ungespannten Nadelschutzfeder abstützt. Während des automatischen Einstechvorgangs der Nadel wird die Nadelschutzfeder gespannt. Nach Abnahme der Injektionsvorrichtung von der Einstichstelle kann die nun gespannte Feder die Nadelschutzhülse distal über die Nadelspitze schieben. Eine Injektionsvorrichtung in der durch Bewegung der Nadelschutzhülse eine Signalfeder zur Erzeugung des "Endklicks" gespannt wird, ist aus EP2489380 bekannt. WO2011/043714 offenbart einen weiteren Mechanismus zur Erzeugung eines "Endklicks".

Es ist eine Aufgabe der Erfindung, einen Autoinjektor anzugeben, der eine Einrichtung zur Erzeugung eines akustischen und/oder taktilen Signals aufweist, wobei der Autoinjektor kostengünstig und betriebssicher ausgestaltet sein soll.

Die Aufgabe wird mit dem Autoinjektor nach Anspruch 1 gelöst. Vorteilhafte Weiterentwicklungen ergeben sich aus den abhängigen Ansprüchen, der Beschreibung und den Figuren.

Der erfindungsgemäße Autoinjektor weist ein Gehäuse und ein in dem Gehäuse angeordneten Produktbehälter auf. Bei dem Produktbehälter kann es sich insbesondere um eine Spritze handeln, welche einen Spritzenkörper aufweist, an dessen distalem Ende eine Injektionsnadel fest angeordnet ist. Der vorzugsweise zylindrische Spritzenkörper umgibt einen Kolben, der in Bezug auf den Spritzenkörper verschiebbar ist und für die Produktausschüttung zu dem distalen Ende hin verschoben wird, wodurch das zwischen dem Kolben und der Injektionsnadel angeordnete flüssige Produkt, insbesondere Medikament, durch die Injektionsnadel aus dem Produktbehälter ausgeschüttet wird. Der Spritzenkörper kann an seinem proximalen, d. h. hinteren oder der Injektionsnadel entgegengesetzten Ende einen Flansch, der auch als Fingerflansch bezeichnet werden kann, aufweisen. Eine derartig ausgebildete Spritze ist als Standardspritze erhältlich, so dass für den Autoinjektor nicht zwingend eine speziell angepasste Spritze entwickelt werden braucht. Der Kolben liegt dichtend an dem Innendurchmesser des Spritzenkörpers an.

Das Gehäuse ist vorzugsweise länglich und bildet die Längsachse des Autoinjektors. Das Gehäuse ist vorzugsweise hülsenförmig und/oder zylindrisch, insbesondere kreiszylindrisch. Der Produktbehälter ist in dem Gehäuse angeordnet. Beispielsweise kann der Behälter verschiebbar in dem Gehäuse angeordnet sein, d. h. für ein automatisches Einstechen relativ zu dem Gehäuse in distale Richtung verschiebbar sein, so dass die Nadelspitze aus einer Öffnung am distalen Ende des Autoinjektors hervortritt und automatisch in den Patienten eingestochen werden kann. Optional kann bei einer solchen Vorrichtung die Nadelspitze nach erfolgter Produktausschüttung in das distale Ende der Vorrichtung hinein bewegt werden, insbesondere der Produktbehälter relativ zu dem Gehäuse in die proximale Richtung bewegt werden.

In bevorzugten Ausführungen ist der Produktbehälter entlang der Längsachse unverschiebbar in dem Gehäuse aufgenommen, insbesondere mittels eines Produktbehälterhalters oder Spritzenhalters, der den Produktbehälter axialfest hält und axialfest mit dem Gehäuse verbunden, insbesondere verrastet ist. Bevorzugt steht die Nadelspitze über das distale Ende des Gehäuses in distale Richtung über. Hierdurch kann die Nadel mittels einer Bewegung des Gehäuses zum Patienten hin in die Einstichstelle eingestochen werden. Vorzugsweise ist eine Nadelschutzhülse vorgesehen, welche das distale Ende des Autoinjektors bildet und eine Öffnung für die Injektionsnadel aufweist, wobei die Nadel durch die Öffnung hindurchtreten kann. Die Nadelschutzhülse kann in ihrer Ausgangsposition in Bezug auf die Nadelspitze so angeordnet sein, dass die Nadelschutzhülse distal über die Nadelspitze steht oder dass die Nadelspitze distal über das distale Ende der Nadelschutzhülse steht. Die Nadelschutzhülse ist relativ zu dem Gehäuse aus ihrer Ausgangsposition in die proximale Richtung um einen Betätigungshub in eine betätigte Position verschiebbar, insbesondere in das Gehäuse verschiebbar, so dass die Nadel aus dem distalen Ende bzw. durch die Öffnung der Nadelschutzhülse hervortritt oder weiter hervortritt. Bevorzugt kann die Nadelschutzhülse um einen Nadelschutzhub aus der betätigten Position relativ zu dem Gehäuse in die distale Richtung in eine Nadelschutzposition verschoben werden, in der das distale Ende der Nadelschutzhülse distal über die Nadelspitze steht, um zu verhindern, dass nach erfolgter Verwendung der Vorrichtung bzw. nach erfolgter Produktausschüttung eine Verletzungsgefahr, die von einer freiliegenden Nadelspitze ausgehen würde, verringert wird. Die Nadelschutzhülse kann z. B. gegen die Kraft einer Feder, die als Nadelschutzfeder bezeichnet werden kann, in die proximale Richtung verschoben werden, wobei die Feder, die z. B. die weiter unten beschriebene zweite Feder oder eine hiervon separate Feder ist, die Nadelschutzhülse aus der betätigten Position in die distale Richtung, d. h. in die Nadelschutzposition verschieben kann. Der Autoinjektor kann ein z. B. federnd angeordnetes Verriegelungsglied aufweisen, welche die Nadelschutzhülse in ihrer Nadelschutzposition insbesondere in Bezug auf das Gehäuse verriegelt und ein Zurückschieben der Nadelschutzhülse in die proximale Richtung oder in das Gehäuse blockiert. Das Verriegelungsglied verriegelt die Nadelschutzhülse zumindest so, dass die Nadel nicht aus dem distalen Ende der Nadelschutzhülse hervortreten kann. Die Nadelschutzhülse kann z. B. aus der Nadelschutzposition nur soweit in die proximale Richtung verschoben werden, dass die Nadelspitze nicht aus dem distalen Ende der Nadelschutzhülse hervortritt.

Der Autoinjektor umfasst ferner ein Vortriebsglied, das zumindest während der Produktausschüttung auf den Kolben wirkt, insbesondere an dem Kolben anliegt, und eine erste Feder, die auf das Vortriebsglied wirkt wie z. B. sich insbesondere mit ihrem distalen Ende an dem Vortriebsglied abstützt. Das Vortriebsglied kann z. B. hülsenförmig sein und eine Schulter, die z. B. im Bereich des distalen Endes des Vortriebsglieds angeordnet ist, bilden, an der sich das distale Ende der ersten Feder abstützen kann. Die erste Feder ist vorzugsweise innerhalb des hülsenförmigen Vortriebsglieds angeordnet. Die Feder ist vorzugsweise eine als Druckfeder wirkende Wendelfeder, die vorzugsweise aus Metall gebildet ist. Die erste Feder ist so stark vorgespannt, insbesondere im Auslieferungszustand des Autoinjektors, dass sie bzw. die in ihr gespeicherte Energie ausreicht, um das Produkt aus dem Produktbehälter durch Verschieben des Vortriebsglieds um einen Ausschütthub im Wesentlichen vollständig auszuschütten. Durch die Verschiebung des Vortriebsglieds um den Ausschütthub wird auch der Kolben verschoben. Sofern im Auslieferungszustand zwischen dem Kolben und dem Vortriebsglied ein Abstand besteht, ist der Ausschütthub des Kolbens kleiner als der Ausschütthub des Vortriebsglieds, was bevorzugt ist, da der Kolben bis zur Verwendung unbelastet bleibt, wodurch eine ungewollte vorzeitige Produktausschüttung vermieden wird. Grundsätzlich ist es aber auch möglich, dass das Vortriebsglied im Auslieferungszustand und nicht erst bei der Produktausschüttung an dem Kolben anliegt. Sofern im Auslieferungszustand das Vortriebsglied an dem Kolben bereits anliegt, entspricht der Ausschütthub des Kolbens dem Ausschütthub des Vortriebsglieds. Das proximale Ende der ersten Feder, die aufgrund ihrer Funktion auch als Ausschüttfeder bezeichnet werden kann, kann sich an dem Gehäuse oder einem gehäusefesten Element, insbesondere auch einem nur zeitweise zu dem Gehäuse axialfesten Element abstützen.

Erfindungsgemäß weist der Autoinjektor ein Signalglied, einen Signalanschlag und eine zweite Feder auf. Die zweite Feder übt eine auf das Signalglied entgegen der Ausschüttrichtung oder in die proximale Richtung wirkende Federkraft aus. Insbesondere kann sich die zweite Feder z. B. mit ihrem proximalen Ende an dem Signalglied abstützen.

Die zweite Feder kann z. B. eine als Druckfeder wirkende Wendelfeder sein, die sich mit ihrem proximalen Ende an dem Signalglied abstützt. Die Feder kann sich mit ihrem distalen Ende z. B. an dem Gehäuse oder einem gehäusefesten Element abstützen. Besonders bevorzugt stützt sich die zweite Feder mit ihrem distalen Ende an der Nadelschutzhülse oder einem Element, welches, insbesondere bei Verschiebung der Nadelschutzhülse relativ zu dem Gehäuse, mit der Nadelschutzhülse verschoben wird, ab. Beispielsweise kann das Element ein Schaltmodul oder eine Schalthülse, wie sie weiter unten beschrieben werden, sein. Das Element kann insbesondere kinematisch und/oder geometrisch zwischen der Nadelschutzhülse und dem distalen Ende der zweiten Feder angeordnet sein. Der Vorteil hierbei ist, dass die Nadelschutzhülse mittels der zweiten Feder aus ihrer betätigten Position in die Nadelschutzposition verschiebbar ist. Die Feder kann somit bevorzugt eine Doppelfunktion erfüllen, da sie zusätzlich die oben genannte Kraft auf das Signalglied ausübt.

Während eines ersten Teilhubs des Ausschütthubs des Vortriebsglieds oder insbesondere im Auslieferungszustand ist das Signalglied axialfest mit dem Vortriebsglied gekoppelt, so dass das Signalglied mit dem Vortriebsglied entlang der Längsachse und insbesondere relativ zu dem Gehäuse insbesondere in die distale Richtung verschiebbar ist. Durch die axialfeste Kopplung mit dem Vortriebsglied wird bewirkt, dass das Signalglied während des Verschiebens des Vortriebsglieds in die Ausschüttrichtung, während der Ausführung des ersten Teilhubs des Ausschütthubs, mitgenommen und die zweite Feder gespannt wird. Während eines zweiten Teilhubs des Ausschütthubs ist die axialfeste Kopplung zwischen dem Signalglied und dem Vortriebsglied gelöst Die axialfeste Kopplung zwischen dem Signalglied und dem Vortriebsglied ist somit lösbar. Wenn die axialfeste Kopplung zwischen dem Signalglied und dem Vortriebsglied gelöst ist - und insbesondere keine weiteren Kopplungen zwischen dem Signalglied und einem weiteren Glied, wie weiter unten beschrieben wird, bestehen - ist das Signalglied mittels der zweiten, vorgespannten Feder entgegen die Ausschüttrichtung und relativ zu dem Vortriebsglied und/oder dem Gehäuse beschleunigbar. Aufgrund der Mitnahme des Signalglieds durch das Vortriebsglied um den ersten Teilhub wird zwischen dem Signalanschlag und dem Signalglied ein z. B. sich entlang der Längsachse erstreckender Abstand, der insbesondere dem ersten Teilhub entspricht, gebildet. Die zweite Feder kann das Signalglied auf diesem Abstand beschleunigen, wodurch das Signalglied mit einer Geschwindigkeit auf den Signalanschlag auftrifft, so dass ein Impuls auf das Signalglied abgegeben wird, der ein akustisches (hörbares) und/oder taktiles (fühlbares) Signal erzeugt.

Der Signalanschlag kann von dem Gehäuse oder einem zumindest axialfest, vorzugsweise auch drehfest mit dem Gehäuse verbundenem Element gebildet werden. Beispielsweise kann dieses Element eine Verschlusskappe am proximalen Ende des Gehäuses sein und/oder das proximale Ende des Autoinjektors bilden. Die Verschlusskappe kann mit dem Gehäuse besonders bevorzugt formschlüssig, alternativ kraft- oder stoffschlüssig verbunden sein. Vorzugsweise ist das Element mit dem Gehäuse verrastet. Eine separate Kappe hat den Vorteil, dass die Montage der Vorrichtung erleichtert wird, wobei für die Montage zumindest ein Teil der Bauteile über das proximale Ende, das danach mit der Kappe verschlossen wird, in das Gehäuse eingebracht werden. Die Kappe kann einen Resonanzkörper bilden, wenn der Signalanschlag an der Kappe angeordnet ist, wobei durch die Gestaltung von Materialdicken und Formen der Kappe der Höreindruck des akustischen Signals in gewissen Grenzen verändert werden kann.

Das Signalglied weist ein erstes, insbesondere federnd oder/und an einem federnden Arm angeordnetes, Eingriffsglied auf, welches in das Vortriebsglied, insbesondere in eine Ausnehmung des Vortriebsglieds lösbar eingreift. Hierdurch wird das Vortriebsglied axialfest mit dem Signalglied gekoppelt, wobei die axialfeste Kopplung zwischen dem Vortriebsglied und dem Signalglied gelöst ist, wenn das Signalglied, insbesondere das erste Eingriffsglied aus dem Eingriff mit dem Vortriebsglied, insbesondere der Ausnehmung des Vortriebsglieds ausgerückt oder herausgedrückt ist. Insbesondere wird das erste Eingriffsglied am Ende des ersten Teilhubs des Vortriebsglieds aus dem Eingriff mit dem Vortriebsglied gelöst.

Bevorzugt ist der Signalanschlag entlang der Längsachse des Autoinjektors so angeordnet, dass er in einer Flucht mit dem Signalglied angeordnet ist. Hierdurch wird erreicht, dass das Signalglied mit einer Bewegung entlang der Längsachse des Autoinjektors an dem Signalanschlag anschlägt.

In Ausführungsformen mit einer Nadelschutzhülse ist es bevorzugt, dass die Nadelschutzhülse auf die zweite Feder wirkt, wobei die Nadelschutzhülse für die Auslösung der Produktausschüttung aus ihrer Ausgangsposition relativ zu dem Gehäuse und entlang der Längsachse des Autoinjektors in die proximale Richtung, d. h. entgegen die Ausschüttrichtung verschiebbar ist, insbesondere um den Betätigungshub. Hierdurch wird die zweite Feder gespannt und auch die Produktausschüttung also die Bewegung des Vortriebsglieds in die Ausschüttrichtung, ausgelöst. Die Nadelschutzhülse wird vorzugsweise dadurch aus ihrer Ausgangsposition um den Betätigungshub in ihre betätigte Position bewegt, dass ihr distales Ende an die Einstichstelle des Patienten angedrückt wird, wobei das Gehäuse relativ zu der Nadelschutzhülse in Richtung Einstichstelle verschoben wird, so dass die Nadelschutzhülse den Betätigungshub relativ zu dem Gehäuse ausführt. Hierbei wird auch die aus dem distalen Ende der Nadelschutzhülse hervortretende Nadel in die Einstichstelle eingestochen. Nach der erfolgten Produktausschüttung, insbesondere nach z. B. einer kurzen Wartezeit, wie z. B. 3 bis 10 Sekunden, nach dem mittels des Signalglieds das Signal erzeugt wurde, wird der Autoinjektor von der Einstichstelle abgenommen, wodurch die Nadelschutzhülse relativ zu dem Gehäuse aus ihrer betätigten Position um den Nadelschutzhub in die Nadelschutzposition verschoben wird, insbesondere mittels der in der zweiten Feder gespeicherten Federenergie. Durch das Abnehmen des Autoinjektors von der Einstichstelle wird auch die Nadel aus der Einstichstelle gezogen.

In bestimmten Ausführungsformen kann kinematisch zwischen der zweiten Feder und der Nadelschutzhülse ein Schaltmodul angeordnet sein, wobei das Schaltmodul von der Nadelschutzhülse in die proximale Richtung mitgenommen wird, wenn die Nadelschutzhülse aus ihrer Ausgangsposition in die proximale Richtung oder in die betätigte Position verschoben wird, und die Nadelschutzhülse in die distale Richtung verschiebt, wenn die auf das Schaltmodul wirkende Feder das Schaltmodul in die distale Richtung verschiebt. Das Schaltmodul oder ein Teil davon, wie z. B. eine Schalthülse, kann mit der Nadelschutzhülse einteilig sein oder z. B. formschlüssig verbunden, wie z. B. verschnappt, sein oder lose an der Nadelschutzhülse anliegen. Das Schaltmodul kann ein einziges Teil sein oder mehrere Teile umfassen, wobei ein mehrteiliges Schaltmodul zumindest die Schalthülse und eine Sperrhülse aufweisen kann. Die Sperrhülse kann relativ zu der Nadelschutzhülse und/oder Schalthülse z. B. entlang der Längsachse verschiebbar sein. Beispielsweise können sich die Feder an der Schalthülse und die Schalthülse an der Nadelschutzhülse abstützen. Zwischen der Sperrhülse und der Schalthülse kann ein z. B. unidirektional wirkendes Verriegelungsglied, welches vorzugsweise das oben genannte Verriegelungsglied, welches die Nadelschutzhülse in ihrer Nadelschutzposition verriegelt, vorgesehen sein, das z. B. von der Sperrhülse gebildet wird und in die Schalthülse, insbesondere in eine Ausnehmung, eingreift. Das Verriegelungsglied ist vorzugsweise so ausgestaltet, dass die Schalthülse während ihrer Bewegung relativ zu dem Gehäuse in die proximale Richtung die Sperrhülse über das Verriegelungsglied mitnimmt, insbesondere während der Bewegung der Nadelschutzhülse aus ihrer Ausgangsposition in die betätigte Position, und während ihrer Bewegung relativ zu dem Gehäuse in die proximale Richtung relativ zu der Sperrhülse in eine Sperrposition verschoben wird, insbesondere während der Verschiebung der Nadelschutzhülse aus ihrer betätigten Position in die Nadelschutzposition, wobei in der Sperrposition das oder ein anderes Verriegelungsglied, wie z. B. das weiter oben genannte, eine Bewegung der Schalthülse relativ zu der Sperrhülse in die proximale Richtung sperrt. Hierdurch wird vorteilhaft verhindert, dass die Nadelschutzhülse aus ihrer Nadelschutzposition für eine erneute Freigabe der Nadelspitze in das Gehäuse zurückgeschoben werden kann.

Z. B. kann die Schalthülse eine erste Ausnehmung aufweisen, in welche das Verriegelungsglied der Sperrhülse lösbar eingreift, wenn die Nadelschutzhülse aus ihrer Ausgangsposition in ihre betätigte Position verschoben wird. Z. B. kann die Schalthülse eine zweite Ausnehmung aufweisen, in welche das Verriegelungsglied oder ggf. das andere Verriegelungsglied eingreift, wenn die Nadelschutzhülse in ihrer Nadelschutzposition ist. Die erste und zweite Ausnehmung können vorzugsweise mit einem Abstand, der in etwa dem Nadelschutzhub entspricht, zueinander entlang der Längsachse angeordnet sein. Selbstverständlich ist auch eine Umkehr der Anordnung der Ausnehmungen und des Verriegelungsglieds oder der Verriegelungsglieder möglich, d. h., dass das zumindest eine Verriegelungsglied an der Schalthülse und die mindestens eine Ausnehmung, d. h. die erste Ausnehmung und ggf. die zweite Ausnehmung an der Sperrhülse gebildet sein können.

Das Verriegelungsglied und ggf. das andere Verriegelungsglied können federnd, insbesondere jeweils an einem federnden Arm, angeordnet sein. Vorzugsweise kann die Schalthülse die Sperrhülse umgeben und/oder führen.

In bevorzugten Ausführungsformen kann das Signalglied ein zweites Eingriffsglied aufweisen, welches durch die Ausrückbewegung des ersten Eingriffsglieds, mit der das erste Eingriffsglied aus dem Vortriebsglied ausrückt, in einen, insbesondere axialfesten, Eingriff mit der Nadelschutzhülse oder dem Schaltmodul, insbesondere der Sperrhülse, bewegbar ist. Das erste Eingriffsglied und das zweite Eingriffsglied sind bevorzugt so aufeinander abgestimmt, dass das zweite Eingriffsglied bereits, vorzugsweise axialfest, in die Nadelschutzhülse oder das Schaltmodul eingreift, wenn das erste Eingriffsglied noch nicht vollständig aus dem Eingriff mit dem Vortriebsglied gelöst ist. Hierdurch wird vorteilhaft zuverlässig verhindert, dass das erste Eingriffsglied bereits aus dem Eingriff mit dem Vortriebsglied gelöst ist, wenn das zweite Eingriffsglied noch nicht in dem Eingriff mit der Nadelschutzhülse oder dem Schaltmodul ist. Die Nadelschutzhülse oder das Schaltmodul, insbesondere die Sperrhülse, kann eine z. B. weitere Ausnehmung aufweisen, in welche das zweite Eingriffsglied des Signalglieds, z. B. für die axialfeste Kopplung zwischen dem Signalglied und dem Schaltmodul, insbesondere Sperrhülse, oder der Nadelschutzhülse, eingreift. Das Vortriebsglied kann eine Ausnehmung aufweisen, in welche das erste Eingriffsglied für die axialfeste Kopplung zwischen Vortriebsglied und Signalglied eingreift. Vorzugsweise sind das erste Eingriffsglied und das zweite Eingriffsglied an einem gemeinsamen elastischen Arm gebildet, wobei das erste Eingriffsglied, z. B. radial, zu der Längsachse hin und das zweite Eingriffsglied, z. B. radial, von der Längsachse weg weisen. Das erste und zweite Eingriffsglied können, vorzugsweise radial zwischen dem Vortriebsglied und der Nadelschutzhülse oder dem Schaltmodul, insbesondere der Sperrhülse angeordnet sein.

Insbesondere werden während des Ausschütthubs des Vortriebsglieds, insbesondere am Ende des ersten Teilhubs, das erste Eingriffsglied aus dem Eingriff mit dem Vortriebsglied gelöst und, vorzugsweise gleichzeitig, das zweite Eingriffsglied in den Eingriff mit dem Schaltmodul oder der Nadelschutzhülse gebracht, insbesondere mit einer Bewegung quer zur Längsachse. Insbesondere kann das Vortriebsglied durch seine Bewegung in die Ausschüttrichtung das erste Eingriffsglied aus der Ausnehmung des Vortriebsglieds und das zweite Eingriffsglied in die Ausnehmung der Nadelschutzhülse oder des Schaltmoduls, insbesondere der Sperrhülse, drücken.

In besonders bevorzugten Ausführungen kann die Nadelschutzhülse oder das Schaltmodul, insbesondere die Sperrhülse, das erste Eingriffsglied in dem Eingriff mit der Ausnehmung des Vortriebsglieds halten, wobei die Ausnehmung für das zweite Eingriffsglied durch Verschieben der Nadelschutzhülse aus ihrer Ausgangsposition in ihre betätigte Position bezogen auf die Längsachse zu dem zweiten Eingriffsglied hin verschoben wird, wobei die Ausnehmung in der betätigten Position der Nadelschutzhülse, insbesondere in dem Moment, an dem der Ausschütthub freigegeben wird, mit einem Abstand entlang der Längsachse, die in etwa dem ersten Teilhub des Signalglieds entspricht, zu dem zweiten Eingriffsglied angeordnet ist. Das für den Ausschütthub durch die Betätigung der Nadelschutzhülse freigegebene Vortriebsglied ist dann um den ersten Teilhub in die Auschüttrichtung bewegbar. Vorzugsweise wird das erste Eingriffsglied in dem Eingriff mit dem Vortriebsglied durch den Innenumfang der Nadelschutzhülse oder des Schaltmoduls, insbesondere der Sperrhülse gehalten, an der bzw. dem das zweite Eingriffsglied anliegt. Das zweite Eingriffsglied befindet sich am Ende des ersten Teilhubs bezogen auf die Längsachse auf der gleichen Position wie die Ausnehmung, wodurch das zweite Eingriffsglied in seine Ausnehmung einrücken und das erste Eingriffsglied aus seiner Ausnehmung ausrücken kann.

Der Ausschütthub des Vortriebsglieds kann insbesondere zwei Phasen umfassen, nämlich den ersten Teilhub und den zweiten Teilhub. Während des ersten Teilhubs ist das erste Eingriffsglied in dem axialfesten Eingriff mit dem Vortriebsglied und das zweite Eingriffsglied aus dem axialfesten Eingriff mit der Nadelschutzhülse oder dem Schaltmodul, insbesondere der Sperrhülse. Während des zweiten Teilhubs des Ausschütthubs ist das zweite Eingriffsglied in dem axialfesten Eingriff mit der Nadelschutzhülse oder dem Schaltmodul, insbesondere der Sperrhülse, wobei das erste Eingriffsglied aus dem Eingriff mit dem Vortriebsglied ist, wodurch vorteilhaft bewirkt wird, dass das Vortriebsglied mittels der ersten Feder relativ zu dem Signalglied in die distale Richtung bewegbar ist und/oder das Signalglied für die Signalauslösung noch nicht gelöst ist.

Allgemein bevorzugt ist, dass das Vortriebsglied mittels der ersten Feder relativ zu dem Signalglied, insbesondere um den zweiten Teilhub, in die distale Richtung bewegbar ist, wenn das erste Eingriffsglied aus dem Eingriff mit dem Vortriebsglied und das zweite Eingriffsglied in dem Eingriff mit der Nadelschutzhülse oder dem Schaltmodul ist.

In bevorzugten Ausführungen können das zweite Eingriffsglied und die Ausnehmung für das zweite Eingriffsglied im Auslieferungszustand des Autoinjektors entlang der Längsachse in etwa mit dem Abstand zueinander angeordnet sein, der in etwa die Summe aus dem Betätigungshub der Nadelschutzhülse und dem ersten Teilhub des Vortriebsglieds, der in etwa dem Hub des Signalglieds von dem Signalanschlag weg entspricht, beträgt.

Bevorzugt kann das Vortriebsglied das zweite Eingriffsglied daran hindern, aus dem axialfesten Eingriff mit der Nadelschutzhülse oder dem Schaltmodul auszurücken, wenn sich das Vortriebsglied relativ zu dem Signalglied in die distale Richtung bewegt, insbesondere während des zweiten Teilhubs des Vortriebsglieds. Das Vortriebsglied erlaubt dem zweiten Eingriffsglied am Ende des Ausschütthubs bzw. des zweiten Teilhubs, dass es aus dem Eingriff mit der Nadelschutzhülse oder dem Schaltmodul ausrückt. Wenn das zweite Eingriffsglied am Ende des zweiten Teilhubs aus dem Eingriff mit der Nadelschutzhülse oder dem Schaltmodul ausgerückt ist, wird das Signalglied von der zweiten Feder entgegen die Ausschüttrichtung beschleunigt und schlägt an dem Signalanschlag an. Vorzugsweise wird das zweite Eingriffsglied in dem Eingriff mit der Nadelschutzhülse oder dem Schaltmodul durch den Außenumfang des Vortriebsglieds gehalten, an dem das erste Eingriffsglied anliegt.

In bevorzugten Ausführungen kann der Autoinjektor ein Halteelement aufweisen, an dem sich z. B. ein Ende der ersten Feder, insbesondere das proximale Ende der ersten Feder, abstützt. Alternativ kann sich die Feder mit ihrem proximalen Ende an dem Gehäuse oder einem gehäusefesten Element abstützen. Das Halteelement selbst kann gehäusefest oder in Bezug auf das Gehäuse verschiebbar angeordnet sein. Das Halteelement kann ein erstes Eingriffselement aufweisen, welches vor dem Auslösen der Produktausschüttung in das Vortriebsglied eingreift, wodurch das Vortriebsglied daran gehindert wird, sich relativ zu dem Halteelement und/oder dem Gehäuse in die Ausschüttrichtung zu bewegen. Der Eingriff des ersten Eingriffselements in das Vortriebsglied ist für die Produktausschüttung lösbar. Wenn der Eingriff gelöst ist, ist das Vortriebsglied für die Bewegung in die Ausschüttrichtung freigegeben. Die erste Feder kann das Vortriebsglied relativ zu dem Halteelement und/oder dem Gehäuse um den Ausschütthub in die Ausschüttrichtung verschieben. Das Vortriebsglied kann eine Ausnehmung für das erste Eingriffselement des Halteelements aufweisen, wobei diese Kopplung zwischen dem Vortriebsglied und dem Halteelement gelöst ist, wenn das Halteelement, insbesondere das erste Eingriffselement, aus dem Eingriff mit dem Vortriebsglied, insbesondere der Ausnehmung des Vortriebsglieds ausgerückt ist. Insbesondere kann das erste Eingriffselement dadurch aus dem Eingriff mit dem Vortriebsglied gelöst werden, dass die Nadelschutzhülse aus der Ausgangsposition um den Betätigungshub in die betätigte Position verschoben wird. Z. B. kann das erste Eingriffselement von der Nadelschutzhülse oder dem Schaltmodul, insbesondere der Sperrhülse in den axialfesten Eingriff mit dem Vortriebsglied gehalten werden, wenn die Nadelschutzhülse nicht in ihrer betätigten Position oder in ihrer Ausgangsposition ist. Z. B. kann ein Innenumfang der Nadelschutzhülse oder des Schaltmoduls, insbesondere der Sperrhülse, das erste Eingriffselement in dem Eingriff mit dem Vortriebsglied halten, wobei z. B. ein zweites Eingriffselement, welches weiter unten beschrieben wird, an dem Innenumfang anliegen kann.

Durch das Verschieben der Nadelschutzhülse in ihre betätigte Position kann die Nadelschutzhülse oder das Schaltmodul, insbesondere die Sperrhülse, dem ersten Eingriffselement erlauben, insbesondere mit einer Bewegung quer zu der Längsachse des Autoinjektors aus dem Eingriff mit dem Vortriebsglied auszurücken. Beispielsweise kann eine Ausnehmung, insbesondere für das zweite Eingriffselement, welche an der Nadelschutzhülse oder dem Schaltmodul, insbesondere der Sperrhülse, gebildet ist, bezogen auf die Längsachse auf der gleichen Position angeordnet sein, wie das erste und/oder zweite Eingriffselement, so dass das erste Eingriffselement aus dem Eingriff mit dem Vortriebsglied ausrücken kann. Beispielsweise kann das Vortriebsglied das erste Eingriffselement aus dem Eingriff mit dem Vortriebsglied drücken, wenn die Nadelschutzhülse in ihrer betätigten Position ist.

Das erste Eingriffselement kann z. B. radial zu der Längsachse hin weisen und/oder an einem federnden Arm des Halteelements angeordnet sein.

Das Halteelement kann - wie erwähnt - ein zweites Eingriffselement aufweisen, welches durch die Ausrückbewegung des ersten Eingriffselements aus dem Vortriebsglied in einen axialfesten Eingriff mit der Nadelschutzhülse oder dem Schaltmodul, insbesondere der Sperrhülse bewegbar ist. Das zweite Eingriffselement kann z. B. an dem Arm, an dem das erste Eingriffselement angeordnet ist, angeordnet sein und/oder z. B. radial von der Längsachse weg weisen. Das erste Eingriffselement und das zweite Eingriffselement können so aufeinander abgestimmt sein, dass das zweite Eingriffselement bereits axialfest in seine Ausnehmung, welche von der Nadelschutzhülse oder dem Schaltmodul, insbesondere der Sperrhülse, gebildet wird, eingreift, wenn das erste Eingriffselement noch nicht vollständig aus dem Eingriff mit dem Vortriebsglied gelöst ist. Hierdurch wird vorteilhaft erreicht, dass zuerst die axialfeste Verbindung zwischen dem Halteelement und der Nadelschutzhülse oder dem Schaltmodul hergestellt ist, bevor die axialfeste Verbindung zwischen dem Halteelement und dem Vortriebsglied gelöst und damit ein erneutes oder weiteres Zurückschieben der Nadelschutzhülse gesperrt wird
Insbesondere wenn das zweite Eingriffselement in seiner Ausnehmung ist, kann sich das Vortriebsglied relativ zu dem Halteelement in distale Richtung bewegen, insbesondere aufgrund der in der vorgespannten Feder gespeicherten Energie. Das Vortriebsglied kann das zweite Eingriffselement daran hindern, aus dem axialfesten Eingriff mit der Nadelschutzhülse oder dem Schaltmodul, insbesondere der Sperrhülse auszurücken, wenn sich das Vortriebsglied relativ zu dem Signalglied in die distale Richtung bewegt. Dies gilt vorzugsweise auch am Ende des Ausschütthubs, insbesondere auch dann, wenn das zweite Eingriffsglied des Signalglieds aus seiner Ausnehmung gelöst ist, um von der zweiten Feder entgegen die Ausschüttrichtung beschleunigt zu werden.

Insbesondere in Ausführungen, in denen die Ausnehmung für das zweite Eingriffselement des Halteelements von der Nadelschutzhülse oder der Schalthülse gebildet wird, ist es bevorzugt, dass das zweite Eingriffselement am Ende des Ausschütthubs aus seiner Ausnehmung ausrückt, um die Nadelschutzhülse nach der Verabreichung des Produkts aus der betätigten Position in die Nadelschutzposition bewegen zu können. Das Vortriebsglied kann hierzu eine Ausnehmung aufweisen, in welche das erste Eingriffselement einrücken kann, wobei das zweite Eingriffselement gleichzeitig aus seiner Ausnehmung ausrückt, um insbesondere die Bewegung der Nadelschutzhülse in distale Richtung freizugeben.

In Ausführungsformen, mit einem Schaltmodul, welches eine Schalthülse und eine Sperrhülse aufweist, ist es bevorzugt, dass das zweite Eingriffselement auch am Ende des Ausschütthubs so verbleibt, dass das zweite Eingriffselement die Sperrhülse daran hindert, relativ zu dem Gehäuse und/oder dem zweiten Eingriffselement in die distale Richtung bewegt zu werden, wobei die Schalthülse und/oder die Nadelschutzhülse relativ zu der Sperrhülse, insbesondere aufgrund der in der zweiten Feder gespeicherten Energie in die distale Richtung verschiebbar sind, wodurch die Nadelschutzhülse insbesondere in ihre Nadelschutzposition bewegt wird. Wie bereits beschrieben und nur der Vollständigkeit halber angemerkt, kann das Verriegelungsglied zwischen der Sperrhülse und der Schalthülse in einen Eingriff gelangen, der verhindert, dass die Schalthülse relativ zu der Sperrhülse in die proximale Richtung verschiebbar ist. Vorzugsweise wird eine Bewegung der Sperrhülse in die proximale Richtung dadurch verhindert, dass die Sperrhülse entweder an dem Gehäuse oder einem gehäusefesten Element, wie z. B. an einem Mechanikhalter, oder dem Signalglied anstößt.

Ein weiterer Aspekt der Erfindung betrifft die Ausgestaltung eines Produktbehälterhalters, insbesondere eines Spitzenhalters für einen Autoinjektor, insbesondere für einen Autoinjektor, bei dem der Produktbehälter in Bezug auf das Gehäuse unverschiebbar ist bzw. für einen Autoinjektor der oben beschriebenen Art.

Die Erfindung geht von einem Spritzenmodul aus, welches insbesondere zur Verwendung in einem Autoinjektor vorgesehen ist. Insbesondere kann ein Autoinjektor vorgesehen sein, der ein solches Spritzenmodul aufweist. Das Spritzenmodul umfasst eine Spritze und einen Spritzenhalter. Die Spritze weist einen Spritzenkörper, einen Kolben und eine Nadel auf, wobei die Nadel z. B. unlösbar an einem Nadelhalteabschnitt der Spritze befestigt ist und der Kolben in einem zylindrischen Abschnitt des Spritzenkörpers verschiebbar angeordnet ist, wobei der Spritzenkörper einen sich verjüngenden Abschnitt oder Bereich aufweist, der zwischen dem Nadelhalteabschnitt und dem zylindrischen Abschnitt angeordnet ist. Die Spritze weist ferner eine Nadelschutzkappe auf, welche z. B. ein sogenanntes soft needle shield oder vorzugsweise ein rigid needle shield sein kann. Ein soft needle shield ist vorzugsweise aus einem gummielastischen Kunststoff gebildet, wobei ein rigid needle shield aus einer Hülse aus Hartkunststoff gebildet ist, in der eine Hülse aus einem gummielastischen Kunststoff angeordnet ist. Die Hülse aus gummielastischem Kunststoff und die Hülse aus Hartkunststoff bilden zusammen das rigid neelde shield. Die Nadelschutzkappe, welche die Nadel abdeckt und an dem sich insbesondere konisch in Richtung Nadelspitze erstreckenden Nadelhalteabschnitt befestigt ist, hält die Nadel vorzugsweise geschützt vor Schmutz und steril. Zwischen dem sich verjüngenden Abschnitt und der Nadelschutzkappe, insbesondere der Hülse aus Hartkunststoff, ist eine Lücke gebildet.

Der Spritzenhalter weist mindestens ein Eingriffsglied, insbesondere eine Schulter, an welcher sich der verjüngende Abschnitt der Spritze in die distale Richtung abstützt und welche in die Lücke zwischen der Nadelschutzkappe und dem sich verjüngenden Abschnitt eingreift, auf. Vorteilhaft wird durch das Anliegen des sich verjüngenden Abschnitts an der mindestens einen Schulter verhindert, dass sich die Spritze relativ zu dem Spritzenhalter in die distale Richtung bewegen kann.

Es ist bevorzugt, dass zwischen der Schulter und der Nadelschutzkappe ein Spalt ist oder besteht, so dass die Nadelschutzkappe von der Schulter unbelastet bleibt. Hierdurch wird vorteilhaft verhindert, dass die Sterilität der Nadel durch ein unbeabsichtigtes Verschieben der Nadelschutzkappe durch die Schulter beeinträchtigt wird.

In bevorzugten Ausführungen kann der Spritzenkörper an seinem proximalen Ende einen Fingerflansch aufweisen, wobei zwischen dem Fingerflansch und dem Spritzenkörper ein Spalt gebildet ist, wenn der sich verjüngende Abschnitt an der Schulter anliegt, wodurch der Fingerflansch im Wesentlichen unbelastet bleibt. Hierdurch wird vorteilhaft verhindert, dass der Fingerflansch überbelastet wird und den Spritzenkörper zerbricht.

Es ist ferner bevorzugt, dass der Spritzenhalter mindestens ein Halteglied, insbesondere eine nach außen gerichtete Abragung aufweist, mit dem der Spritzenhalter axialfest mit einem Gehäuse des Autoinjektors verbindbar oder verbunden ist, insbesondere verschnappt oder verschnappbar ist.

Insbesondere kann der Spritzenhalter mindestens einen Nocken aufweisen, der federnd, insbesondere an einem Arm angeordnet ist und z. B. distal des Halteglieds angeordnet ist. Der mindestens eine Nocken kann eine Nadelschutzhülse an der Bewegung aus ihrer Ausgangsposition in ihre betätigte Position so hemmen oder hindern, dass beim Überschreiten einer auf die Nadelschutzhülse entlang der Längsachse L des Autoinjektors ausgeübten Grenzkraft der mindestens eine Nocken aus dem Eingriff mit der Nadelschutzhülse gedrückt wird, wodurch die Nadelschutzhülse schlagartig in ihre betätigte Position relativ zu dem Gehäuse verschiebbar ist.

Das Gehäuse des Autoinjektors kann z. B. einen Halteabschnitt aufweisen, der an dem Spritzenhalter, insbesondere an einer Außenfläche oder einem Außenumfang des Spritzenhalters anliegt und das mindestens eine Eingriffsglied daran hindert, sich quer zur Längsachse von der Längsachse weg zu bewegen. Insbesondere kann der Halteabschnitt ringförmig sein und das mindestens eine Eingriffsglied, vorzugsweise zwei oder drei oder vier Eingriffsglieder umgeben, so dass das mindestens eine Eingriffsglied innerhalb des Halteabschnitts angeordnet ist. Für die Montage bzw. das Einlegen der Spritze in den Spritzenhalter befindet sich der Spritzenhalter außerhalb des Eingriffs mit dem Halteabschnitt des Gehäuses. Wenn die Spritze vollständig in den Spritzenhalter eingelegt ist, insbesondere das mindestens eine Eingriffsglied in die Lücke zwischen dem sich verjüngenden Abschnitt und der Nadelschutzkappe eingreift, wird das Spritzenmodul bzw. der Spritzenhalter in den Eingriff mit dem Halteabschnitt gebracht, so dass verhindert wird, dass sich das mindestens eine Eingriffsglied aus dem Eingriff mit dem verjüngenden Abschnitt quer zur Längsachse, insbesondere von der Längsachse weg oder nach außen, bewegt.

In einer ersten Variante kann das mindestens eine Eingriffsglied federnd, insbesondere an einem Arm an dem Spritzenhalter gebildet sein, wobei die Spritze über das proximale Ende mit der Nadel voraus in den Spritzenhalter, der vorzugsweise hülsenförmig ist, eingeschoben wird, wobei die Nadelschutzkappe das mindestens eine Eingriffsglied quer zur Längsachse nach außen, d. h. von der Längsachse weg auslenkt, wobei, wenn die Nadelschutzkappe vollständig an dem mindestens einen Eingriffsglied vorbei bewegt wurde, das mindestens eine Eingriffsglied in die Lücke zwischen dem sich verjüngenden Bereich und der Nadelschutzkappe einschnappt. Anschließend wird der Spritzenhalter mit der Spritze in den Eingriff mit dem Halteabschnitt des Gehäuses des Autoinjektors verschoben, wodurch das mindestens eine Eingriffsglied in den Eingriff mit der Lücke zwischen der Nadelschutzkappe und dem sich verjüngenden Abschnitt gehalten wird und aus diesem Eingriff nicht mehr herausfedern kann.

In weiteren Varianten kann der Spritzenhalter mindestens zwei Schalenkörper, insbesondere zwei Halbschalen, umfassen, von denen vorzugsweise jeder ein solches Eingriffsglied aufweist, wobei das Eingriffsglied starr, d. h. im Wesentlichen unbeweglich an dem Schalenkörper angeordnet sein kann. Durch Zusammenfügen der mindestens zwei Schalenkörper kann das mindestens eine Eingriffsglied in die Lücke zwischen die Nadelschutzkappe und dem sich verjüngenden Abschnitt der zwischen den Schalenkörpern angeordneten Spritze eingefügt werden, wodurch die Spritze gegen eine Bewegung in die distale Richtung blockiert wird.

Insbesondere können zwei Schalenkörper über ein Schwenkgelenk verbunden sein, wobei die Schalenkörper in Bezug zueinander aus einer Einfügeposition, in welcher die Spritze in den Spritzenhalter einlegbar ist, in eine Schließposition schwenkbar sein, in welcher das mindestens eine Eingriffsglied in die Lücke zwischen der Nadelschutzkappe und dem sich verjüngenden Bereich eingreift. Die Schalenkörper können in der Schließposition miteinander verrasten oder verrastet sein oder alternativ lose anliegen, wobei die Schalenkörper durch den Halteabschnitt zusammengehalten werden können.

Da die Schalenkörper vorzugsweise aus Kunststoff, wie z. B. transparentem Kunststoff gebildet sind, kann das Schwenkgelenk ein Filmscharnier sein, so dass der erste und zweite Schalenkörper über das Filmscharnier einstückig verbunden sind.

Alternativ kann das Schwenkgelenk ein Scharnier sein, welches mindestens einen Scharnierzapfen und mindestens eine Scharnierzapfenaufnahme aufweist, in welcher der Scharnierzapfen angeordnet, insbesondere verrastet ist und relativ zu welcher der Scharnierzapfen schwenkbar ist oder/und an welcher der Scharnierzapfen beim Verschwenken gleitet. Beispielsweise kann der erste Schalenkörper zwei Scharnierzapfen und der zweite Schalenkörper zwei Scharnierzapfenaufnahmen für die zwei Scharnierzapfen aufweisen. Vorzugsweise weist der erste Schalenkörper jeweils einen Scharnierzapfen und eine Scharnierzapfenaufnahme auf, wobei der zweite Schalenkörper ebenfalls jeweils einen Scharnierzapfen und eine Scharnierzapfenaufnahme aufweist, wobei der Scharnierzapfen des einen Schalenkörpers in die Scharnierzapfenaufnahme des anderen Schalenkörpers eingefügt ist oder werden kann. Der Vorteil hierbei ist, dass der erste Schalenkörper und der zweite Schalenkörper identisch gebildet und auf Umschlag zusammengefügt werden können, so dass die Werkzeugkosten für die Herstellung verringert werden können.

Die Schwenkachse des Schwenkgelenks kann parallel oder quer, insbesondere senkrecht oder wird schief auf die Längsachse der Spritze, insbesondere der Injektionsnadel der Spritze sein. Wenn die Schwenkachse parallel zu der Längsachse ist, ist es bevorzugt, dass die Schwenkachse seitlich des Spritzenkörpers angeordnet ist. Wenn die Schwenkachse quer zu der Längsachse angeordnet ist, ist es bevorzugt, dass das Scharnier bzw. die Schwenkachse an dem proximalen Ende des Spritzenhalters angeordnet ist.

In bevorzugten Ausführungen können der erste und zweite Schalenkörper miteinander durch eine Linearbewegung quer zur Längsachse zusammengefügt und das mindestens eine Eingriffsglied beim Zusammenfügen in die Lücken zwischen der Nadelschutzkappe und dem sich verjüngenden Bereich des Spritzenkörpers bewegt werden, insbesondere mittels der linearen Fügebewegung. Die ersten und zweiten Schalenkörper können in Bezug auf den Spritzendurchmesser auf gegenüberliegenden Seiten jeweils verrastet oder verschnappt sein, alternativ lose aneinander anliegen, wobei der Halteabschnitt des Gehäuses des Autoinjektors die Schalenkörper zusammenhalten kann.

Bevorzugt sind der erste Schalenkörper und der zweite Schalenkörper in einer Einfügeposition, in der die Spritze in den Spritzenkörper einlegbar ist, mittels mindestens einer, vorzugsweise mehreren Sollbruchstellen verbunden, wobei die mindestens eine Sollbruchstelle durch Gegeneinanderdrücken des ersten und zweiten Schalenkörpers zerbrochen wird, wobei der erste und zweite Schalenkörper in ihre Schließposition gebracht werden, in der das mindestens eine Eingriffsglied in die Lücke zwischen der Nadelschutzkappe und dem sich verjüngenden Bereich eingreift. Vor dem Zerbrechen der mindestens einen Sollbruchstelle können der erste und zweite Schalenkörper einstückig miteinander verbunden sein, wobei ein hülsenförmiger Körper gebildet wird, über dessen proximales Ende die Spritze, insbesondere mit der Nadel oder Nadelschutzkappe voran eingefügt werden kann.

Allgemein bevorzugt können der erste Schalenkörper und der zweite Schalenkörper jeweils die mindestens eine Abragung für die Verbindung mit dem Gehäuse und/oder dem Nocken für die Hemmung beim Zurückschieben der Nadelschutzhülse aufweisen.

In weiteren Ausführungen kann der erste Schalenkörper das mindestens eine Eingriffsglied und eine seitliche Öffnung aufweisen, durch die die Spritze seitlich in den ersten Schalenkörper einfügbar ist, wobei beim seitlichen Einfügen der Spritze in den Spritzenkörper das mindestens eine Eingriffsglied in die Lücke zwischen der Nadelschutzkappe und dem sich verjüngenden Abschnitt eingefügt wird. Beispielsweise kann der erste Schalenkörper die mindestens eine Abragung und/oder den mindestens einen Nocken aufweisen.

Vorzugsweise weist der Spritzenhalter einen zweiten, insbesondere hülsenförmigen Schalenkörper auf, in welchen der erste Schalenkörper zusammen mit der Spritze und einer Bewegung entlang der Längsachse eingefügt werden kann, insbesondere über das proximale Ende des zweiten Schalenkörpers und vorzugsweise mit der Nadelspitze oder der Nadelschutzkappe voran. In der Ausführung mit dem zweiten Schalenkörper kann optional der zweite Schalenkörper die mindestens eine Abragung und/oder den mindestens einen Nocken aufweisen.

Bevorzugt kann der zweite Schalenkörper einen Translationsanschlag aufweisen, an dem ein Translationsgegenanschlag des ersten Schalenkörpers anliegt, so dass eine Verschiebung des ersten, in dem zweiten Schalenkörper aufgenommenen Schalenkörpers relativ zum zweiten Schalenkörper in die distale Richtung verhindert wird, d. h. insbesondere wenn der erste Schalenkörper in den zweiten Schalenkörper eingefügt ist. Der Halteabschnitt des Gehäuses kann bevorzugt so ausgestaltet sein, dass das mindestens eine Eingriffsglied des ersten Schalenkörpers in dem Eingriff mit dem sich verjüngenden Bereich hält und vorzugsweise auch an dem Bereich des zweiten Schalenkörpers anliegt, in dem der Translationsanschlag gebildet ist.

In weiteren bevorzugten Ausführungen kann der Spritzenhalter mindestens einen ersten hülsenförmigen Schalenkörper, insbesondere einen hülsenförmigen Grundkörper, und mindestens einen, vorzugsweise zwei Schwenkhebel aufweisen, wobei an dem ersten Schalenkörper der Schwenkhebel mittels eines Scharniers um eine Schwenkachse schwenkbar angeordnet ist. Die Schwenkachse kann sich vorzugsweise quer zur Längsrichtung der Spritze erstrecken, vorzugsweise windschief in Bezug auf die Längsachse der Spritze angeordnet sein. An dem Schwenkhebel, bei mehreren Schwenkhebeln, vorzugsweise an jedem der mehreren Schwenkhebel kann das Eingriffsglied angeordnet sein, insbesondere am von der Schwenkachse entfernten Ende des Schwenkhebels. Vorzugsweise ist das Eingriffsglied distal des Schwenkgelenks angeordnet. Das Schwenkgelenk kann ein Filmscharnier sein, welches den Schwenkhebel und den ersten hülsenförmigen Schalenkörper verbindet. Alternativ kann das Schwenkgelenk ein Scharnier sein, welches mindestens einen Scharnierzapfen und mindestens eine Scharnierzapfenaufnahme aufweist, in welcher der Scharnierzapfen angeordnet, insbesondere verrastet ist und relativ zu welcher der Scharnierzapfen schwenkbar ist oder/und an welcher der Scharnierzapfen beim Verschwenken gleitet. Vorzugsweise kann der Schwenkhebel zwei Scharnierzapfen und der Hülsenkörper zwei Scharnierzapfenaufnahmen für die Scharnierzapfen des Schwenkhebels aufweisen, oder umgekehrt. Wenn mehrere Schwenkhebel vorhanden sind, können entsprechend mehrere solcher Schwenkzapfenaufnahmen vorhanden sein.

Vorzugsweise kann der Halteabschnitt des Gehäuses so auf den mindestens einen Schwenkhebel wirken, insbesondere an dem mindestens einen Schwenkhebel anliegen, dass das Eingriffsglied in dem Eingriff mit dem sich verjüngenden Bereich des Spritzenkörpers gehalten wird. Beim Einfügen der Spritze über das proximale Ende des Hülsenkörpers mit der Nadel bzw. der Nadelschutzkappe voran, wird das mindestens eine Eingriffsglied nach außen geschwenkt, wodurch die Nadelschutzkappe an dem mindestens einen Eingriffsglied vorbeibewegt werden kann, so dass das mindestens eine Eingriffsglied in die Lücke eingreifen kann, wenn die Lücke in Bezug auf die Längsachse auf der Position des mindestens einen Eingriffsglieds ist.

Vorzugsweise kann an dem Schwenkhebel der Nocken für die Nadelschutzhülse gebildet sein. Insbesondere kann der Schwenkhebel ein zweiarmiger Schwenkhebel sein, wobei an einem Arm, der vorzugsweise in die proximale Richtung von dem Schwenkgelenk abragt, der Nocken gebildet ist, wobei an dem anderen Arm, der vorzugsweise in die distale Richtung von dem Schwenkgelenk abragt, das mindestens eine Eingriffsglied gebildet ist. Wenn der Halteabschnitt des Gehäuses den Schwenkhebel in dem Eingriff mit dem sich verjüngenden Bereich hält, kann der Schwenkarm, an dem sich der Nocken befindet, beim Zurückschieben der Nadelschutzhülse elastisch verformt werden.

Die Erfindung und der weitere Aspekt zur Erfindung wurden anhand mehrerer bevorzugter Ausführungen beschrieben. Im Folgenden werden besonders bevorzugte Ausführungen anhand von Figuren beschrieben. Die dabei offenbarten Merkmale bilden den Gegenstand der Erfindung einzeln und in jeglicher Merkmalskombination vorteilhaft weiter. Es zeigen:
- Figur 1: eine Explosionsdarstellung eines Autoinjektors gemäß einer besonders bevorzugten Ausführungsform,
- Figuren 2a-2c: der Autoinjektor aus Figur 1 in einem Auslieferungszustand, wobei die Figuren 2a bis 2c durch die Längsachse der Vorrichtung verlaufende Schnittansichten sind, wobei die Schnittansichten um die Längsachse winkelversetzt sind,
- Figuren 3a-3c: die Vorrichtung und die Ansichten aus den Figuren 2a-2c, wobei sich eine Nadelschutzhülse in ihrer betätigten Position befindet,
- Figuren 4a-4c: die Vorrichtung und die Ansichten aus den Figuren 2a-2c, wobei das Vortriebsglied am Ende eines ersten Teilhubs seines Ausschütthubs gezeigt wird,
- Figuren 5a-5c: die Vorrichtung und die Ansichten aus den Figuren 2a-2c, wobei ein Vortriebsglied am Ende seines Ausschütthubs gezeigt wird,
- Figuren 6a-6c: die Vorrichtung und die Ansichten aus den Figuren 2a-2c, wobei ein Signal, welches das Ende der Produktausschüttung signalisiert, erzeugt wird,
- Figuren 7a-7c: die Vorrichtung und die Ansichten aus den Figuren 2a-2c, wobei die Nadelschutzhülse in ihrer Nadelschutzposition ist,
- Figuren 8a-8d: perspektivische Ansichten eines mehrteiligen Spritzenhalters gemäß einer ersten Variante,
- Figuren 9a-9c: perspektivische Ansichten eines Spritzenhalters gemäß einer zweiten Variante,
- Figuren 10a-10d: perspektivische Ansichten eines Spritzenhalters gemäß einer dritten Variante und
- Figuren 11a-11c: perspektivische Ansichten eines Spritzenhalters gemäß einer vierten Variante.
- Figuren 12a-12c: perspektivische Ansichten eines Spritzenhalters gemäß einer fünften Variante.

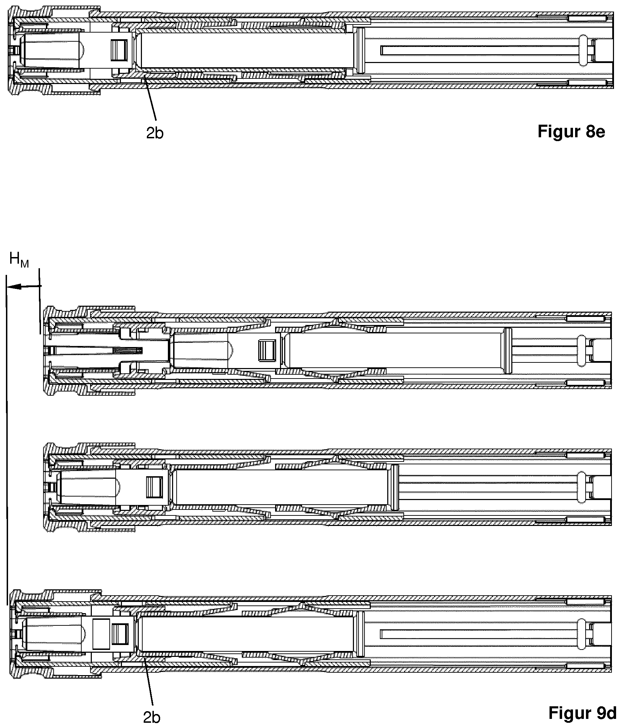

- Figuren 8e, 9d, 10e, 11d, 12d: Längsschnitte der fünf Ausführungsformen im Auslieferungszustand und für die Ausführungsformen zwei bis fünf in je einer Position mit teilweise und vollständig eingesetzter Spritze

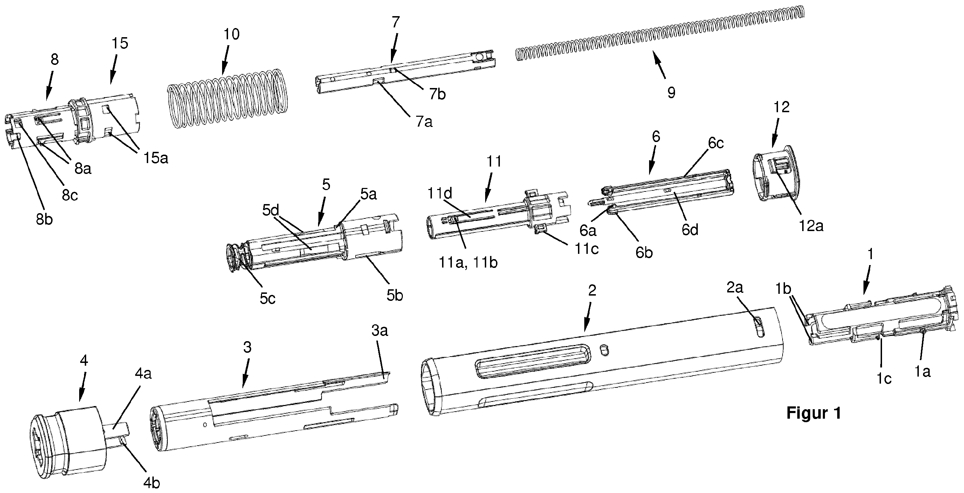

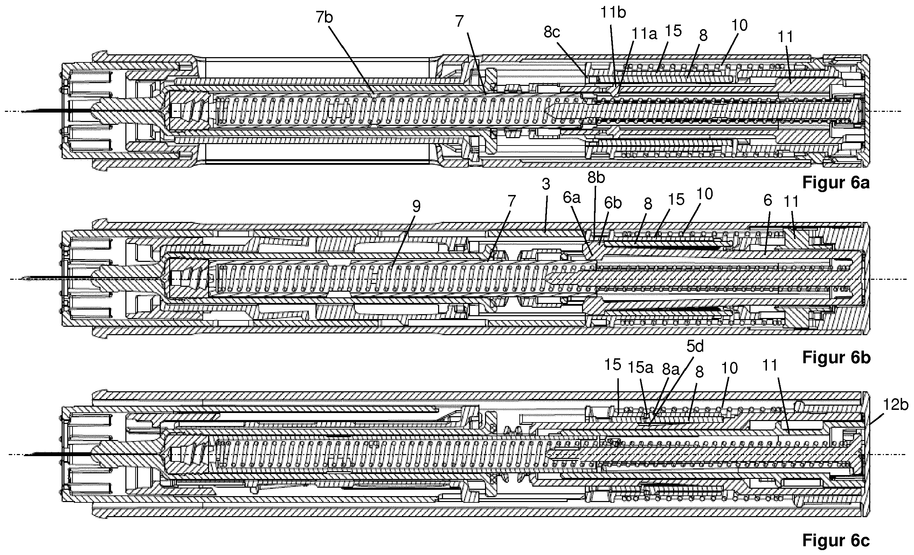

Bezugnehmend auf die Figuren 1-7c werden nun die strukturellen Merkmale und die Funktion des bevorzugten Autoinjektors beschrieben.

Der Autoinjektor weist ein hülsenförmiges, längliches Gehäuse 2 mit einer Längsachse L auf, welches an seinem proximalen Ende eine Verschlusskappe 12 aufweist, die formschlüssig mit dem Gehäuse 2 dreh- und axialfest verbunden ist und das proximale Ende des Autoinjektors bildet. Die Verschlusskappe 12 ist mit dem Gehäuse 2 verschnappt. Hierfür weist die Verschlusskappe 12 ein Rastglied 12a auf, welches in eine Ausnehmung 2a am Gehäuse 2 eingerastet ist, vorzugsweise so, dass die Verschlusskappe 12 nicht oder nicht ohne weiteres von dem Gehäuse 2 lösbar ist.

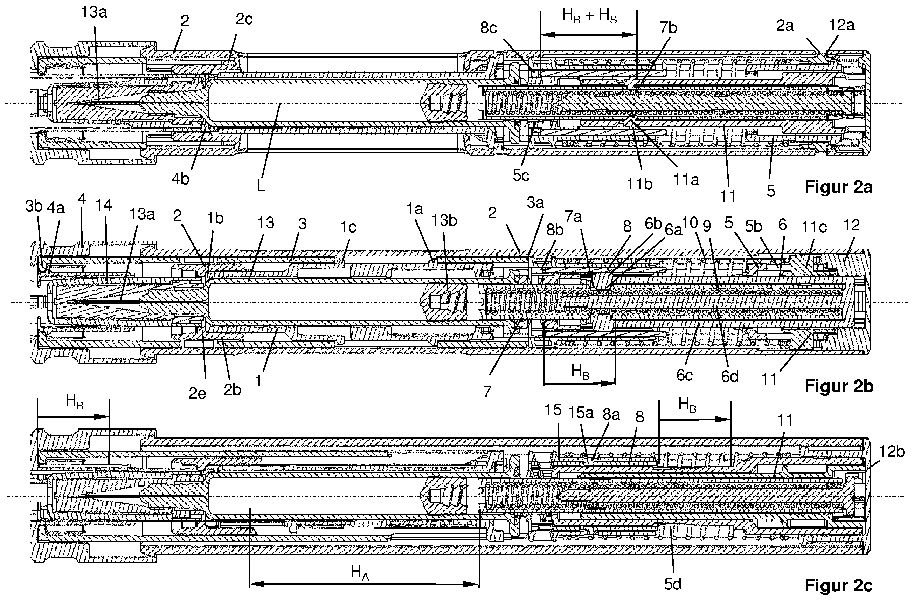

An dem distalen Ende des Autoinjektors ist in seinem Auslieferungszustand (Figuren 2a-2c) eine Abziehkappe 4 angeordnet, die vor der Verwendung des Autoinjektors abgezogen, oder abgedreht, und entfernt wird.

In dem Gehäuse 2 ist ein Produktbehälter 13 in der Gestalt einer Spritze in Bezug auf das Gehäuse 2 - abgesehen von der Montage des Autoinjektors - entlang der Längsachse L unverschiebbar aufgenommen. Der Produktbehälter 13 weist einen hülsenförmigen Spritzenkörper auf, der einen Kolben 13b, der dichtend an dem Innenumfang des Spritzenkörpers anliegt, umgibt. Der Spritzenkörper weist an seinem distalen Ende eine insbesondere unlösbar mit dem Spritzenkörper verbundene Injektionsnadel 13a auf, deren distales Ende von der Nadelspitze gebildet wird. Zwischen der Injektionsnadel 13a und dem Kolben 13b ist ein flüssiges Produkt, insbesondere Medikament, innerhalb des Spritzenkörpers angeordnet, wobei das flüssige Produkt durch Verschieben des Kolbens 13b in eine Ausschüttrichtung, d. h. in distale Richtung oder zu der Injektionsnadel 13a hin das Produkt durch die hohle Injektionsnadel 13a aus dem Produktbehälter 13 ausschüttet. Der Spritzenkörper weist an seinem proximalen Ende einen sogenannten Fingerflansch auf, der radial nach außen über den Außenumfang des zylindrischen Spritzenkörpers ragt.

Der Produktbehälter 13 ist in einem Produktbehälterhalter, der als Spritzenhalter 1 bezeichnet wird, so aufgenommen, dass er zumindest gegen eine Bewegung entlang der Längsachse L in distale Richtung relativ zu dem Spritzenhalter 1 gesichert ist. Der Spritzenhalter 1 ist, wie am besten aus Figur 2a ersichtlich ist, mit dem Gehäuse 2 formschlüssig verbunden, insbesondere verrastet. Das Gehäuse 2 weist hierzu Ausnehmungen auf, in die Rastelemente, die hier am proximalen Ende des Spritzenhalters 1 gebildet werden, eingreifen. Der Spritzenhalter 1 weist mindestens eine nach innen ragende Schulter 1b auf, an der sich ein verjüngender Abschnitt des Produktbehälters 13, der distal des zylindrischen Spritzenkörperabschnitts, welcher den Kolben 13b führt, abstützt.

Um zu verhindern, dass der Produktbehälter 13 relativ zu dem Spritzenhalter 1 in die proximale Richtung verschiebbar ist, wird der Produktbehälter 13 an seinem proximalen Ende durch einen auf den Spritzenkörper wirkenden Halter in den Eingriff mit der Schulter 1b gedrückt. Der Halter wird von einem Haltefederabschnitt 5c eines Mechanikhalters 5 gebildet. Der Mechanikhalter 5 ist in Bezug auf das Gehäuse 2 entlang der Längsachse L insbesondere unverschiebbar und/oder drehfest angeordnet. Der hülsenförmige Mechanikhalter 5 kann mit dem Gehäuse 2 verschnappt sein. Durch den Haltefederabschnitt 5c können Längenunterschiede des Produktbehälters 13, die aufgrund von Herstellungstoleranzen entstehen können, ausgeglichen werden, wobei der feste Sitz des Produktbehälters 13 an der Schulter 1b sichergestellt ist.

Der Produktbehälter 13 ist in Bezug auf das Gehäuse 2 so angeordnet, dass die Nadelspitze distal über das distale Ende des Gehäuses 2 übersteht. Im Ausgangs- oder Auslieferungszustand des Autoinjektors, d. h., wenn die Abziehkappe 4 an dem Autoinjektor angeordnet ist, wird die Nadel 13a von einer Nadelabdeckkappe 14, die in dem gezeigten Beispiel als sogenanntes und dem Fachmann bekanntes rigid needle shield, alternativ als soft needle shield, ausgebildet ist, abgedeckt, um die Nadel 13a vor Verschmutzung zu schützen bzw. die Nadel 13a und das Medikament steril zu halten. Das rigid needle shield 14 ist an einem Nadelhalteabschnitt des Spritzenkörpers angeordnet, wobei sich der verjüngende Abschnitt des Spritzenkörpers zwischen dem Nadelhalteabschnitt und dem zylindrischen Abschnitt des Spritzenkörpers befindet. Die Schulter 1b ist zwischen dem Spritzenkörper und dem proximalen Ende des rigid needle shield 14 angeordnet, insbesondere so, dass zwischen dem rigid needle shield 14 und der Schulter 1b ein - wenngleich auch geringer - Spalt entsteht, um zu verhindern, dass die Schulter 1b eine Kraft auf das rigid needle shield 14 ausübt, wodurch z. B. die Sterilität der Nadel 13a oder des flüssigen Produkts gefährdet werden könnte. Die Abziehkappe 4 ist mit dem Gehäuse 2 oder einer Nadelschutzhülse 3 lösbar verschnappt, wobei diese Verschnappung gelöst wird, wenn die Abziehkappe 4 von dem Gehäuse 2 oder der Nadelschutzhülse 3 entfernt wird. Die Verschnappung wird in dem gezeigten Beispiel durch eine Schnappgeometrie 3b der Nadelschutzhülse 3 und einem Schnapphaken 4a der Abziehkappe 4 gebildet (Figur 2b). Diese Schnapphaken 4a sichern die Abziehkappe 4 weiter gegen eine proximale Bewegung relativ zum Gehäuse 2 indem sie auf dem Gehäuse 2 oder an einer distalen Stirnseite an dem Spritzenhalter 1 eine gehäusefeste Abstützung finden. Die Abziehkappe 4 weist ferner insbesondere an einem Schnapphaken 4a mindestens einen Schnapper 4b auf, der in eine Lücke zwischen dem Spritzenkörper, insbesondere dessen sich verjüngenden Bereichs, und dem proximalen Ende des rigid needle shield 14 eingreift. Wenn die Abziehkappe 4 von dem Autoinjektor entfernt wird, hakt der Schnapper 4b in das proximale Ende des rigid needle shield 14 ein, wodurch das rigid needle shield 14 von dem Produktbehälter 13 gelöst und zusammen mit der Abdeckkappe 4 von dem Autoinjektor entfernt wird.

Der Autoinjektor weist eine Nadelschutzhülse 3 auf, die relativ zu dem Gehäuse 2 und entlang der Längsachse L um einen Betätigungshub H_{B} in die proximale Richtung in eine betätigte Position verschiebbar ist, um eine Produktausschüttung auszulösen. In der Ausgangsposition der Nadelschutzhülse 3, wie sie in den Figuren 2a-2c gezeigt wird, wobei die Abziehkappe 4 abgenommen ist, steht das distale Ende der Nadelschutzhülse 3 distal über die Nadelspitze der Nadel 13a über, so dass ein Zugriff auf die Nadelspitze zunächst verhindert wird. Durch Verschieben der Nadelschutzhülse 3 um den Betätigungshub H_{B} wird die Nadelschutzhülse 3 soweit in die proximale Richtung verschoben, dass die Nadel 13a aus dem distalen Ende der Nadelschutzhülse 3 hervortritt, insbesondere mit einer Länge hervortritt, welche der Injektionstiefe der Nadel in die Einstichstelle entspricht. Bevorzugt soll die Nadel 13a soweit über das distale Ende der Nadelschutzhülse 3 überstehen, dass eine subkutane Injektion erfolgen kann. Insbesondere kann das Gehäuse 2 einen Anschlag 2c bilden, an dem die Nadelschutzhülse 3 in der betätigten Position anliegt.

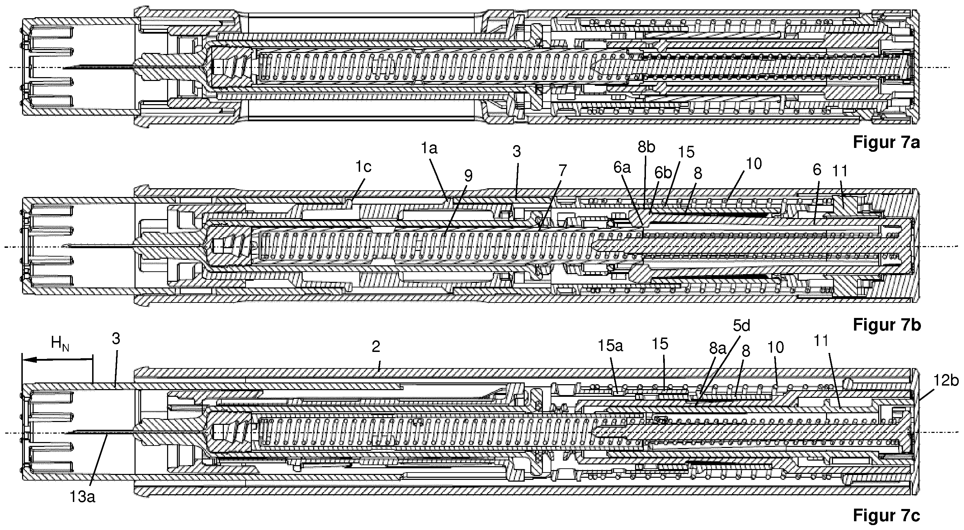

Nach der erfolgten Injektion kann die Nadelschutzhülse 3 relativ zu dem Gehäuse 2 aus der betätigten Position entlang der Längsachse L um einen Nadelschutzhub H_{N} in die distale Richtung in eine Nadelschutzposition (Figuren 7a-7c) verschoben werden. In der Nadelschutzposition steht das distale Ende der Nadelschutzhülse 3 distal über die Nadelspitze über, so dass ein Zugriff auf die Nadelspitze verhindert und eine Verletzungsgefahr verringert wird. Die Nadelschutzhülse 3 kann, wie weiter unten beschrieben wird, gegen erneutes Zurückschieben aus der Nadelschutzposition blockiert werden.

Der Spitzenhalter 1 weist eine Abragung 1a, die radial nach außen weist, auf, wobei die Abragung 1a in eine schlitzförmige Ausnehmung der Nadelschutzhülse 3, die zwischen dem Gehäuse 2 und dem Spritzenhalter 1 angeordnet ist, eingreift. In der Ausgangsposition der Nadelschutzhülse 3 (Figuren 2a-2c) und/oder in der Nadelschutzposition der Nadelschutzhülse 3 (Figuren 7a-7c) liegt die Nadelschutzhülse 3, insbesondere das proximale Ende der schlitzförmigen Ausnehmung an der Abragung 1a an, wodurch eine Bewegung der Nadelschutzhülse 3 in die distale Richtung verhindert wird. In diese schlitzförmige Ausnehmung, alternativ in eine andere Ausnehmung der Nadelschutzhülse 3, kann ein Nocken 1c, der federnd an dem Spritzenhalter 1 angeordnet und von dem Spritzenhalter 1 gebildet ist, eingreifen. Der Nocken 1c ist so gestaltet, dass bei dem Versuch, die Nadelschutzhülse 3 aus der Ausgangsposition in die betätigte Position zu verschieben, der Nocken 1c die Verschiebung der Nadelschutzhülse 3 zunächst verhindert, wobei der Nocken 1c herausgedrückt wird, wenn die auf die Nadelschutzhülse 3 zum Zurückschieben aufgebrachte Kraft einen gewissen Schwellwert überschreitet, wodurch die Nadelschutzhülse 3 schlagartig in die betätigte Position zurückgeschoben wird. Die Nadel 13a kann hierdurch schlagartig in die Einstichstelle eingestochen werden. Zum Einstechen der Nadel 13a bzw. zum Verschieben der Nadelschutzhülse 3 in die betätigte Position wird das distale Ende der Nadelschutzhülse 3 in die Einstichstelle angesetzt, wobei das Gehäuse 2 dann in Richtung Einstichstelle gedrückt wird, wobei wenn die Andrückkraft den oben genannten Schwellwert überschreitet, das Gehäuse 2 schlagartig zu der Einstichstelle hin und die Nadelschutzhülse 3 relativ zu dem Gehäuse 2 in die betätigte Position verschoben wird.

Das Gehäuse 2 weist einen ringförmigen Halteabschnitt oder Ringabschnitt 2b auf, der insbesondere das distale Ende des Spritzenhalters 1 insbesondere ringförmig umgibt, und daran anliegt, wodurch die mindestens eine Schulter 1b in dem Eingriff mit dem verjüngenden Bereich des Spritzenkörpers gehalten wird. Ferner weist das Gehäuse 2 im Bereich des Halteabschnitts 2b einen Translationsanschlag in der Gestalt einer Halteschulter 2e auf, die verhindert, dass der Spritzenhalter 1 relativ zu dem Gehäuse 2 in die distale Richtung verschiebbar ist, wenn der Spritzenhalter 1 an der Halteschulter 2e anliegt. Dies gilt auch vorteilhaft für die beschriebenen Varianten.

Der Autoinjektor weist ferner ein hülsenförmiges Vortriebsglied 7 auf, welches an seinem distalen Ende eine nach innen ragende Schulter bildet, an der sich eine erste Feder 9, die auch als Ausschüttfeder bezeichnet werden kann, abstützt. Die erste Feder 9 ist innerhalb des hülsenförmigen Vortriebsglieds 7 angeordnet. Die erste Feder 9 ist eine als Druckfeder wirkende Wendelfeder, die im Ausgangs- oder Auslieferungszustand des Autoinjektors mit so viel Energie vorgespannt ist, dass sie das in dem Produktbehälter 13 enthaltene Produkt insbesondere vollständig durch Verschieben des Vortriebsglieds 7 um einen Ausschütthub H_{A} aus dem Produktbehälter 13 ausschütten kann. Im Auslieferungszustand der Vorrichtung besteht zwischen dem Kolben 13b und dem distalen Ende des Vortriebsglieds 7 ein Abstand, so dass das Vortriebsglied 7 erst während der Ausführung des Ausschütthubs H_{A} an den Kolben 13b anschlägt und diesen in die Ausschüttrichtung mitnimmt.

Die erste Feder 9 stützt sich mit ihrem proximalen Ende an einem Halteelement 6 ab, welches in diesem Beispiel zwei Arme 6c aufweist, wobei an jedem Arm 6c ein erstes Eingriffselement 6a und ein zweites Eingriffselement 6b angeordnet ist. Das erste Eingriffselement 6a weist radial zu der Längsachse L hin, wobei das zweite Eingriffselement 6b radial von der Längsachse L weg weist. Das erste Eingriffselement 6a greift in eine erste Ausnehmung 7a, die von dem Vortriebselement 7 gebildet wird, ein, wodurch eine Bewegung des Vortriebsglieds 7 relativ zu dem Halteelement 6 in die distale Richtung oder in die Ausschüttrichtung verhindert wird. Hierdurch wird die erste Feder 9 in ihrem gespannten Zustand gehalten. Das Halteelement 6 weist einen Führungsstift 6d auf, der durch das proximale Ende der ersten Feder 9 in die Seele der Feder 9 eingefügt ist. Der Führungsstift 6d verhindert ein seitliches Ausknicken der ersten Feder 9 während und am Ende des Ausschütthubs H_{A} des Vortriebsglieds 7.

Der Autoinjektor weist ein Schaltmodul 8, 15 auf, welches eine Schalthülse 15 und eine von der Schalthülse 15 umgebene Sperrhülse 8 aufweist. Im Auslieferungszustand der Vorrichtung wird das erste Eingriffselement 6a von dem Innenumfang der Sperrhülse 8, der an dem zweiten Eingriffselement 6b anliegt, in dem Eingriff mit der ersten Ausnehmung 7a gehalten.

Die Schalthülse 15 ist mit dem proximalen Ende 3a der Nadelschutzhülse 3 verbunden oder liegt zumindest an dem proximalen Ende 3a der Nadelschutzhülse 3 an. Eine zweite Feder 10, innerhalb der die erste Feder 9 angeordnet ist und die vorzugsweise die Schalthülse 15 und die Sperrhülse 8 zumindest teilweise umgibt, stützt sich mit ihrem distalen Ende an der Schalthülse 15 ab. Ein Teil der Schalthülse 15 ist somit zwischen der Nadelschutzhülse 3 und dem distalen Ende der zweiten Feder 10 angeordnet. Die zweite Feder 10 ist eine als Druckfeder wirkende und als Wendelfeder ausgestaltete Feder aus Metall. Die zweite Feder 10 stützt sich mit ihrem proximalen Ende an einem Signalglied 11, insbesondere an einer Abragung 11c, die axial verschiebbar und verdrehfest in das Gehäuse 2 eingreift und die durch eine schlitzförmige Nut 5b des Mechanikhalters 5 hindurchgreift, ab. Die zweite Feder 10 umgibt somit auch den Mechanikhalter 4 zumindest teilweise, vorzugsweise vollständig.

Das Schaltglied 15 weist eine Ausnehmung 15a auf, in welche ein Verriegelungsglied 8a der Sperrhülse 8 eingreift. Das Verriegelungsglied 8a ist sägezahnförmig und ragt radial von der Längsachse L weg. Das Verriegelungsglied 8a ist federnd an einem Arm, welcher von der Sperrhülse 8 gebildet wird, angeordnet. Durch Verschieben der Schalthülse 15 in die proximale Richtung wird die Sperrhülse 8 über den Eingriff des Verriegelungsglieds 8a in die proximale Richtung mitgenommen.

Durch Verschieben der Nadelschutzhülse 3 in die betätigte Position wird die Schalthülse 15 ebenfalls um den Betätigungshub H_{B} mitgenommen, wodurch die zweite Feder 10 gespannt wird. Wird die Nadelschutzhülse 3 nicht vollständig in die betätigte Position verschoben, kann die zweite Feder 10 die Schalthülse 15 und die Nadelschutzhülse 3 wieder zurück in die Ausgangsposition verschieben, wobei über den Eingriff des Verriegelungsglieds 8a auch die Sperrhülse 8 von der Schalthülse 15 mitgenommen wird.

Das insbesondere hülsenförmige Signalglied 11 ist im Auslieferungszustand oder vor der Auslösung der Produktausschüttung in einem axialfesten Eingriff mit dem Vortriebsglied 7. Das Signalglied 11 weist ein erstes Eingriffsglied 11a, welches in eine Ausnehmung 7b des Vortriebsglieds 7 eingreift, und ein zweites Eingriffsglied 11b auf. Das erste Eingriffsglied 11a und das zweite Eingriffsglied 11b sind an dem Ende eines Arms 11d federnd angeordnet. Das Signalglied 11 weist zwei solcher Arme 11d mit einem ersten Eingriffsglied 11a und einem zweiten Eingriffsglied 11b auf. Das erste Eingriffsglied 11a weist radial zu der Längsachse L hin, wobei das zweite Eingriffsglied 11b radial von der Längsachse L weg weist. Im Auslieferungszustand wird das erste Eingriffsglied 11a von dem Innenumfang der Sperrhülse 8 in dem axialfesten Eingriff mit dem Vortriebsglied 7 gehalten. Das zweite Eingriffsglied 11b liegt an dem Innenumfang der Schalthülse 8 an. Die Verschlusskappe 12 weist einen Signalanschlag 12b auf, an den das Signalglied 11 zur Erzeugung eines Signals anschlagen kann und vorzugsweise an dem das Signalglied 11 im Auslieferungszustand der Vorrichtung anliegt.

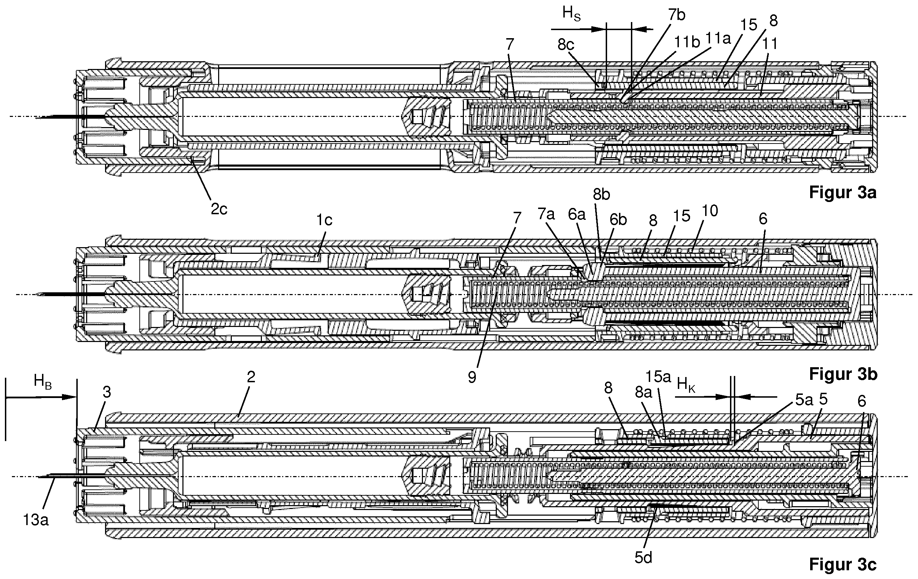

Zur Verabreichung des Produkts aus dem Produktbehälter 13 wird die Abziehkappe 4 von dem Autoinjektor zusammen mit dem rigid needle shield 14 entfernt. Das distale Ende der Nadelschutzhülse 3 wird an die Einstichstelle eines Patienten angesetzt, wobei das Gehäuse 2 zu der Einstichstelle hin verschoben wird, wodurch sich die Nadelschutzhülse 3 aus ihrer Ausgangsposition um den Betätigungshub H_{B} in die proximale Richtung relativ zu dem Gehäuse 2 in die betätigte Position bewegt. Hierdurch wird die zweite Feder 10 gespannt, wobei die Schalthülse 15 von der Nadelschutzhülse 3 um den Betätigungshub H_{B} mitgenommen wird. Die Sperrhülse 8 weist eine erste Ausnehmung 8b auf, die durch Verschieben der Sperrhülse 8 um den Betätigungshub H_{B} entlang der Längsachse L auf die Position des zweiten Eingriffselements 6b gebracht wird, wie in den Figuren 3a-3c dargestellt. Hierdurch wird das erste Eingriffselement 6a aus dem Eingriff mit dem Vortriebsglied 7 mit einer Bewegung quer zu und von der Längsachse L weg bewegt, wobei gleichzeitig das zweite Eingriffselement 6b in den Eingriff mit der Sperrhülse 8, insbesondere deren erster Ausnehmung 8b bewegt wird. Hierdurch wird das Vortriebsglied 7 für die Bewegung um den Ausschütthub H_{A} in die Ausschüttrichtung freigegeben.

Da die axialfeste Kopplung zwischen dem Vortriebsglied 7 und dem Halteelement 6 nun aufgehoben ist, kann das Halteelement 6, welches zumindest ein Stück weit relativ zu dem Gehäuse 2 und entlang der Längsachse L bewegbar ist, von der ersten Feder 9 in die proximale Richtung bewegt werden, wobei das Halteelement 6 über den Eingriff des zweiten Eingriffselements 6b in die Ausnehmung 8b die Sperrhülse 8 um einen Startsignalhub H_{K} (Figur 3c) mitnimmt, wodurch die Sperrhülse 8 an einem Startsignalanschlag 5a, der von dem Mechanikhalter 5 gebildet wird, anschlägt und hierdurch ein akustisches und/oder taktiles Signal ausgibt, welches dem Anwender der Vorrichtung signalisiert, dass die Produktausschüttung gestartet wurde. Durch die Verschiebung der Sperrhülse 8 um den Betätigungshub H_{B} wird das Verriegelungsglied 8a für eine Bewegung quer und zu der Längsachse L hin freigegeben, da der Mechanikhalter 5 eine Vertiefung 5d aufweist, welche eine solche Bewegung des Verriegelungsglieds 8a zulässt, wenn die Sperrhülse 8 um den Betätigungshub H_{B} verschoben wurde oder wenn die Nadelschutzhülse 3 in ihrer betätigten Position ist.

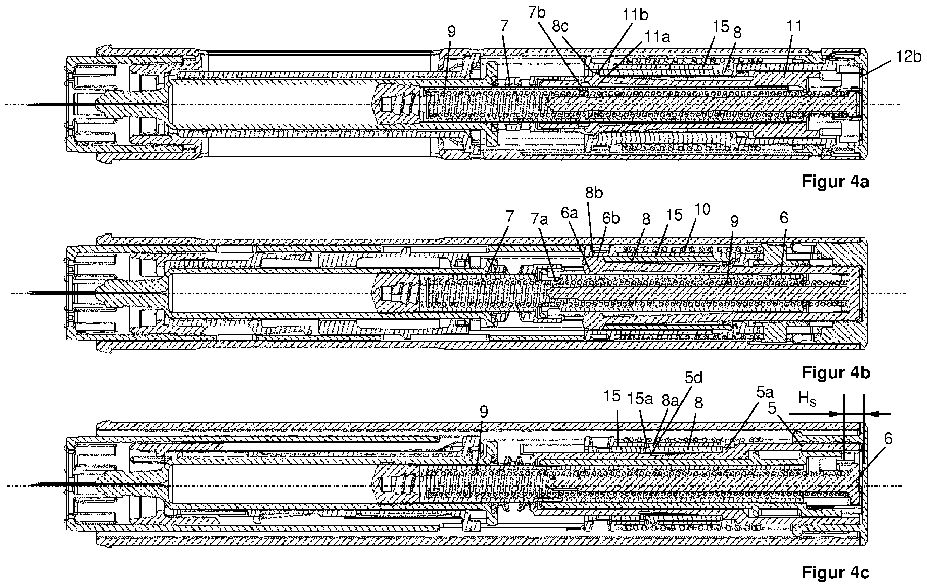

Da das Signalglied 11 noch axialfest mit dem Vortriebsglied 7 verbunden ist, wird es um einen ersten Teilhub H_{S} des Ausschütthubs H_{A} in die Ausschüttrichtung mitgenommen, wobei das Signalglied 11 in etwa um den ersten Teilhub H_{S} von dem Signalanschlag 12b weg bewegt wird, wie am besten aus Figur 4c erkennbar ist. Am Ende des ersten Teilhubs H_{S}, während dem das erste und zweite Eingriffsglied 11a, 11b relativ zu der Sperrhülse 8 bewegt werden, wird das erste Eingriffsglied 11a aus dem Eingriff mit dem Vortriebsglied 7 gedrückt, wobei gleichzeitig das zweite Eingriffsglied 11b in die zweite Ausnehmung 8c der Sperrhülse 8 mit einer Bewegung quer zu der Längsachse L und radial von der Längsachse L weg bewegt wird. Hierdurch wird das Signalglied 11 daran gehindert, sich in die proximale Richtung relativ zu dem Gehäuse 2 oder der Sperrhülse 8 zu bewegen. Das zweite Eingriffsglied 11b wird von dem Außenumfang des Vortriebsglieds 7 in den Eingriff mit der Ausnehmung 8c gehalten (Figur 4a), wenn das Vortriebsglied 7 um seinen zweiten Teilhub des Ausschütthubs H_{A} bewegt wird. Die Außenumfangsfläche des Vortriebsglieds 7 hält das zweite Eingriffselement 6b in dem Eingriff mit der ersten Ausnehmung 8b der Sperrhülse 8, wie am besten aus Figur 4b erkennbar ist. Am Ende des Ausschütthubs H_{A} gibt das Vortriebsglied 7 das zweite Eingriffsglied 11b aus dem Eingriff mit der Sperrhülse 8 frei, wodurch das zweite Eingriffsglied 11b aus dem Eingriff mit der Ausnehmung 8c bewegt wird, insbesondere zu der Längsachse L hin, so dass die zweite Feder 10 das Signalglied 11 entgegen die Ausschüttrichtung, d. h. in die proximale Richtung beschleunigt, so dass beim Auftreffen des Signalglieds 11 auf dem Signalanschlag 12b ein akustisches und/oder taktiles Signal erzeugt wird.

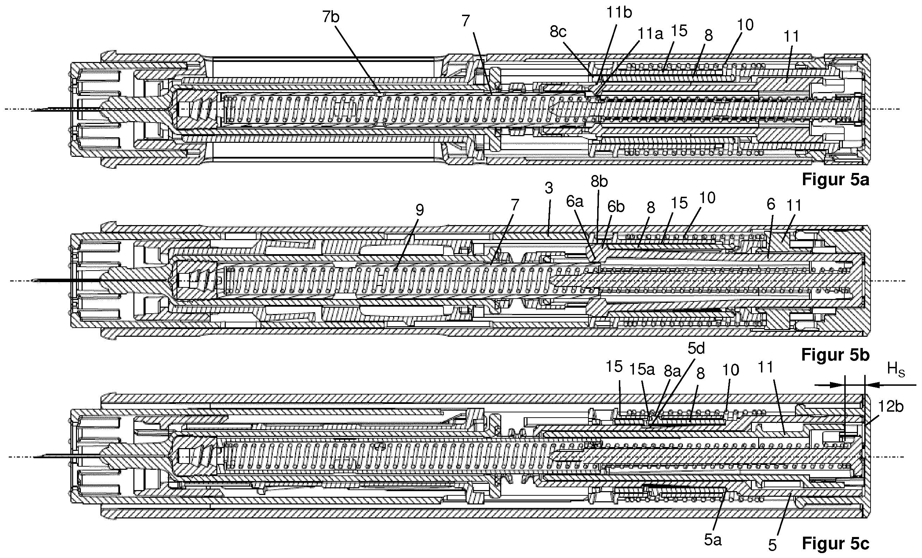

Wie am besten aus Figur 5b erkennbar ist, bleibt der Eingriff des zweiten Eingriffselements 6b in die erste Ausnehmung 8b bestehen, wodurch eine Bewegung der Sperrhülse 8 in die distale Richtung relativ zu dem Gehäuse 2 verhindert wird.

Durch Abnehmen des Autoinjektors von der Injektionsstelle kann die zweite Feder 10 die Schalthülse 15 und die Nadelschutzhülse 3 aus der betätigten Position in die Nadelschutzposition um den Nadelschutzhub H_{N} bewegen, wobei das Verriegelungsglied 8a aus dem Eingriff mit der Ausnehmung 15a gedrückt wird, wobei sich die Schalthülse 15 relativ zu der Sperrhülse 8 in die distale Richtung bewegt. Wenn die Nadelschutzhülse 3 in ihrer Nadelschutzposition ist, verschnappt das Verriegelungsglied 8a mit der Schalthülse 15, wobei das Verriegelungsglied 8a ein Zurückschieben der Nadelschutzhülse 3 in ihre betätigte Position verhindert. Bei dem Versuch, die Nadelschutzhülse 3 aus der Nadelschutzposition in die betätigte Position zurückzuschieben, stößt das Schaltglied 15 an dem Verriegelungsglied 8a an, welches die Bewegung der Nadelschutzhülse 3 in die betätigte Position verhindert. Die Sperrhülse 8 stützt sich hierzu axial an dem Startsignalanschlag 5a des Mechanikhalters 5 ab.

Im Folgenden werden verschiedene Ausführungen eines Spritzenhalters gezeigt, die bei einem Autoinjektor, vorzugsweise aber nicht notwendigerweise einem Autoinjektor der oben beschriebenen Art, eingesetzt werden können.

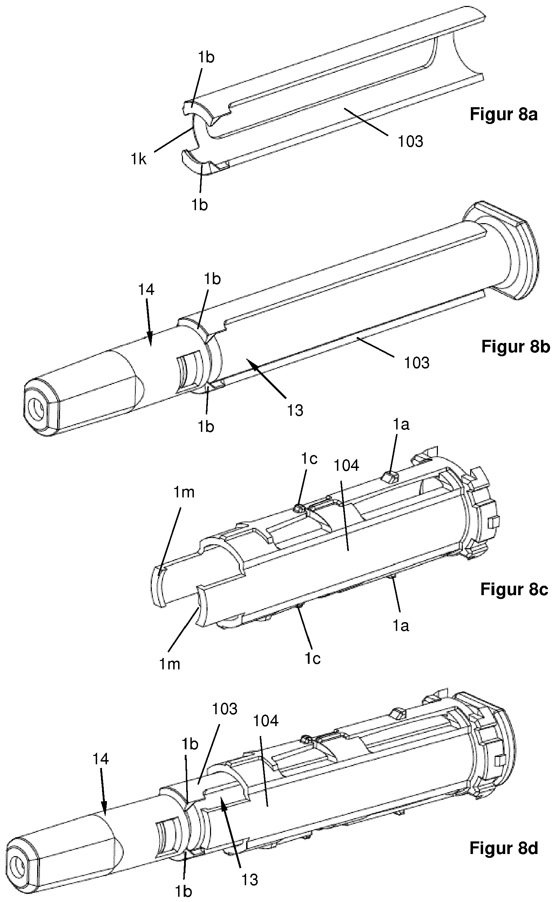

Das Spritzenmodul aus den Figuren 8a bis 8d weist einen ersten Schalenkörper oder Hülsenkörper 103 auf, der seitlich eine Öffnung aufweist und mindestens eine, d. h. in dem gezeigten Beispiel zwei schulterförmige Eingriffsglieder 1b aufweist, die nach innen, d. h. zur Längsachse des Hülsenkörpers 103 ragen. Ferner weist der Hülsenkörper 103 einen in die distale Richtung weisenden Translationsgegenanschlag 1k auf. Für die Montage der Spritze 13 (Figur 8b) wird diese seitlich, d. h. mit einer Bewegung quer zur Längsachse in den Hülsenkörper 103 eingefügt, wodurch das mindestens eine Eingriffsglied 1b in die Lücke zwischen der Nadelschutzkappe 14 und dem sich verjüngenden Abschnitt des Spritzenkörpers der Spritze 13 eingefügt werden.

Das Spritzenmodul weist ferner einen zweiten Schalenkörper, insbesondere Hülsenkörper 104 auf (Figur 8c), der an seinem proximalen Ende offen ist und an seinem distalen Ende mindestens einen, d. h. in dem gezeigten Beispiel zwei Translationsanschläge 1m aufweist, die radial nach innen abragen. Wie in der Ausführung aus den Figuren 1 bis 7c weist der Hülsenkörper 104 mindestens einen Nocken 1c, nämlich zwei Nocken 1c und mindestens eine Abragung 1a, nämlich zwei Abragungen 1a auf. Der Nocken 1c ist über einen Arm federnd an dem Hülsenkörper 104 angeordnet.

Die Einheit aus Spritze 13, Nadelschutzkappe 14 und dem ersten Hülsenkörper 103 wird über das proximale Ende entlang der Längsachse mit der Nadelschutzkappe 14 voran (Figur 8b) in den zweiten Hülsenkörper 104 (Figur 8c) eingeschoben, wobei der Translationsgegenanschlag 1k an den Translationsanschlag 1m anschlägt, wenn die Einheit 13, 14, 103 vollständig in den Hülsenkörper 104 eingeschoben ist (Figur 8d). Die in Figur 8d gezeigte Einheit wird dann für die Montage so in dem Gehäuse 2 des Autoinjektors verschoben, dass der Halteabschnitt 2b, insbesondere der ringförmige Halteabschnitt oder Ringabschnitt, an zumindest dem ersten Hülsenkörper 103 zumindest im Bereich des Eingriffsglieds 1b anliegt, so dass das Eingriffsglied 1b in dem Eingriff mit dem sich verjüngenden Abschnitt des Spritzenkörpers gehalten wird. Ferner kann der Halteabschnitt 2b auch an dem zweiten Hülsenkörper 104 anliegen, insbesondere in dem Bereich, an dem der mindestens eine Translationsanschlag 1m gebildet ist, um den Translationsanschlag 1m im Eingriff mit dem Translationsgegenanschlag 1k zu halten.

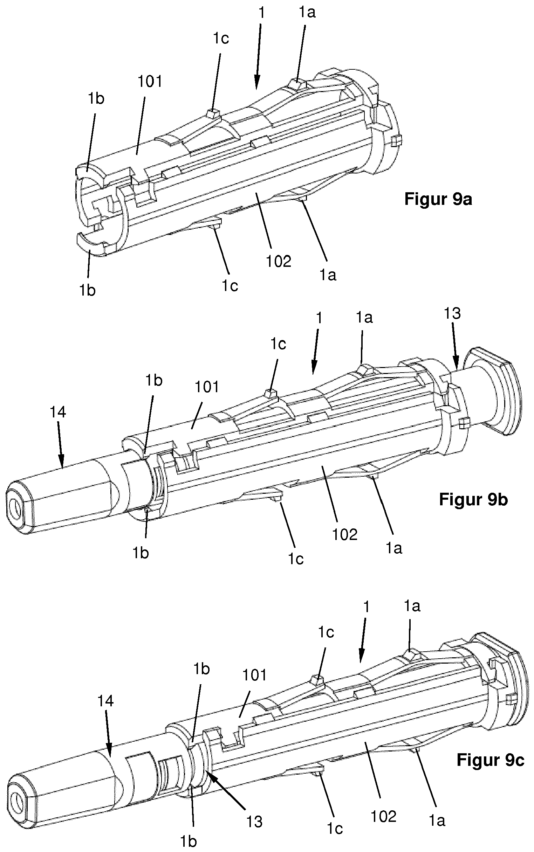

In der in den Figuren 9a-9c gezeigten Ausführungsform weist das Spritzenmodul, insbesondere der Spritzenhalter einen ersten Schalenkörper 101 und einen zweiten Schalenkörper 102 auf, die jeweils als Halbschale gebildet sind. Jeder der Schalenkörper 101, 102 weist einen Nocken 1c und eine Abragung 1a in der hierin beschrieben Weise auf.

Der erste Schalenkörper 101 und der zweite Schalenkörper 102 sind in der in Figur 9a gezeigten Ansicht über mehrere Sollbruchstellen einteilig miteinander verbunden, wobei der erste und zweite Schalenkörper 101, 102 eine Einfügeposition zueinander einnehmen. Über das proximale Ende des in Figur 9a gezeigten Körpers 101, 102 wird die Spritze 13 mit der Nadelschutzkappe 14 voran in die distale Richtung eingeschoben (Figur 9b) bis die Lücke zwischen dem sich verjüngenden Abschnitt und der Nadelschutzkappe 14 entlang der Längsachse L auf der gleichen Position angeordnet ist wie das mindestens eine Eingriffsglied 1b. In dem gezeigten Beispiel weist jeder aus erstem und zweitem Schalenkörper 101, 102 ein Eingriffsglied 1b auf. Durch Gegeneinanderdrücken der ersten und zweiten Schalenkörper 101, 102 quer zur Längsachse L werden die Sollbruchstellen zerbrochen, wobei der erste und zweite Schalenkörper 101, 102 formschlüssig miteinander verrasten und die Eingriffsglieder 1b in die Lücke bewegt werden. Wie bereits beschrieben können der Bereich des ersten und zweiten Schalenkörpers 101, 102, an dem das Eingriffsglied 1b gebildet ist, von dem Halteabschnitt 2b des Gehäuses 2 eingefasst werden, wodurch die Eingriffsglieder 1b in dem Eingriff mit dem sich verjüngenden Bereich des Spritzenkörpers gehalten werden. Besonders bevorzugt können sich die Schalenkörper 101, 102 beim Einschieben der Spritze 13 quer zur Längsachse gegen die elastische Kraft der Arme welche die Abragung 1a und/oder den Nocken 1c tragen in die Einfügeposition bewegen. Wie beschrieben können anschliessend auch hier die Eingriffsglieder 1b von dem Halteabschnitt 2b des Gehäuses 2 in Eingriff mit dem sich verjüngenden Abschnitt des Spritzenkörpers 13 gebracht und gehalten werden. Alternativ oder zusätzlich können der erste Schalenkörper 101 und der zweite Schalenkörper 102 in der Schließposition (Figur 9c), in der die Eingriffsglieder 1b in die Lücke eingreifen, miteinander verrasten.

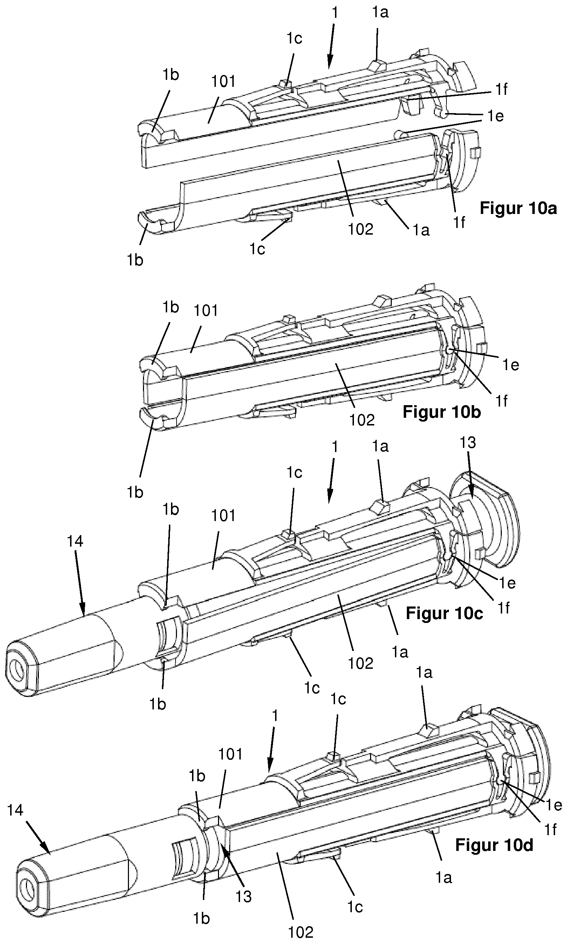

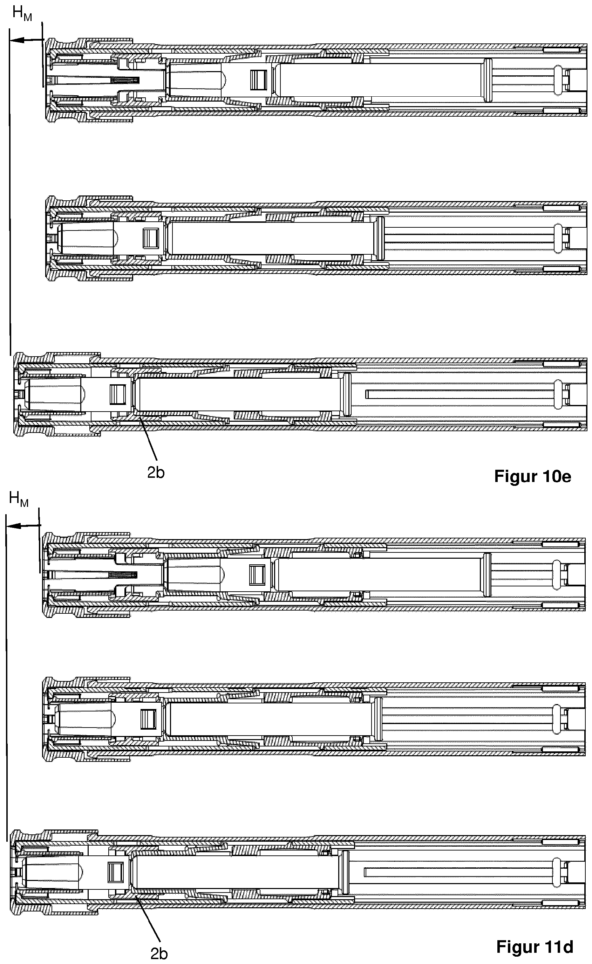

In der in den Figuren 10a-10d gezeigten Ausführung weist der Spritzenhalter 1 einen ersten Schalenkörper 101 und einen zweiten Schalenkörper 102 auf, die jeweils als Halbschale gebildet sind und insbesondere identisch ausgestaltet sind, damit Werkzeugkosten gespart werden können.

Jeder der ersten und zweiten Schalenkörper 101, 102 weist einen Nocken 1c und eine Abragung 1a in der beschriebenen Weise auf. Ferner weist jeder der ersten und zweiten Schalenkörper 101, 102 an seinem distalen Ende ein Eingriffsglied 1b auf.

Jeder der Schalenkörper 101, 102 weist einen Scharnierzapfen 1e und eine Scharnierzapfenaufnahme 1f auf (Figur 10a), wobei der Scharnierzapfen le des einen Schalenkörpers 101, 102 in die Scharnierzapfenaufnahme 1f des anderen Schalenkörpers 102, 101 eingefügt wird (Figur 10b), so dass die erste und zweite Halbschale 101, 102 relativ zueinander um die Schwenkachse des Schwenkgelenks 1e, 1f, welches aus den Scharnierzapfen le und den Scharnierzapfenaufnahmen 1f gebildet wird, schwenkbar sind, nämlich zwischen einer Einfügeposition (Figur 10c) und einer Schließposition (Figur 10d). Über das proximale Ende des Spritzenkörpers 1 wird die Spritze 13 zusammen mit der Nadelschutzkappe 14 eingefügt, wobei die Nadelschutzkappe 14 an dem Eingriffsglied 1b vorbeibewegt wird, wobei der erste Schalenkörper 101 und der zweite Schalenkörper 102 zueinander verschwenkt werden, wenn die Lücke zwischen der Nadelschutzkappe 14 und dem sich verjüngenden Bereich des Spritzenkörpers in Bezug auf die Längsachse L auf der gleichen Position wie die Eingriffsglieder 1b sind. Hierdurch greifen die Eingriffsglieder 1b in die genannte Lücke. Wie beschrieben können die Eingriffsglieder 1b von dem Halteabschnitt 2b des Gehäuses 2 in Eingriff mit dem sich verjüngenden Abschnitt des Spritzenkörpers gehalten werden. Alternativ oder zusätzlich können der erste Schalenkörper 101 und der zweite Schalenkörper 102 in der Schließposition (Figur 10d), in der die Eingriffsglieder 1b in die Lücke eingreifen, miteinander verrasten.

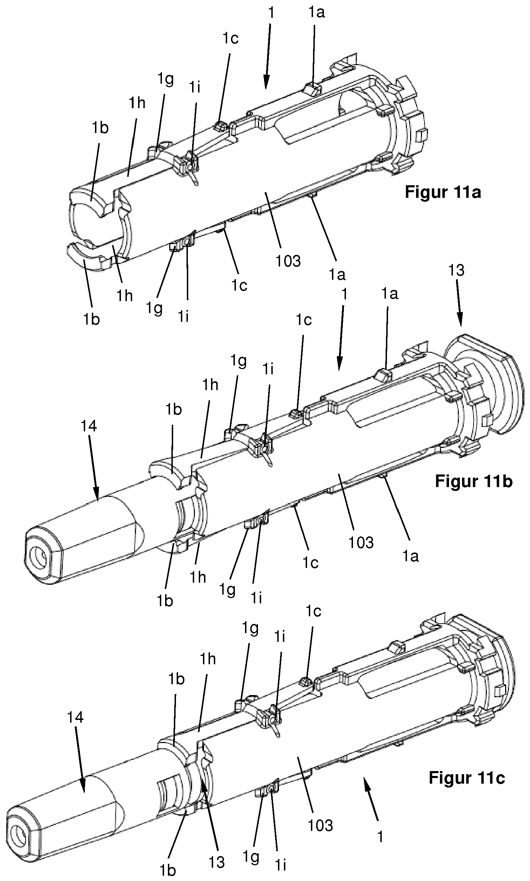

In den Figuren 11a bis 11c wird eine Ausführungsform des Spritzenhalters 1 gezeigt, die einen ersten Hülsenkörper 103 und zwei Schwenkarme 1h aufweist. Die Abragung 1a ist an dem Hülsenkörper 103 gebildet. Der Hülsenkörper 103 bildet für jeden der Schwenkarme 1h zwei Scharnierzapfenaufnahmen 1g, in denen jeweils ein Scharnierzapfen 1i des Schwenkhebels 1h angeordnet ist. Jeder der Schwenkhebel 1h bildet zwei Scharnierzapfen 1i, die mit der Scharnierzapfenaufnahme verrastet sind. Der Schwenkzapfen 1i ist relativ zu der Schwenkzapfenaufnahme 1g drehbar und kann an der Scharnierzapfenaufnahme 1g gleiten. Der Schwenkhebel 1h weist einen Hebelabschnitt auf, der in die distale Richtung weist, wobei am distalen Ende dieses Hebelabschnitts das vom Schwenkhebel 1h gebildete Eingriffsglied 1b für den Eingriff in die Lücke zwischen der Nadelschutzkappe 14 und dem sich verjüngenden Abschnitt des Spritzenkörpers gebildet ist.

Der in dem Beispiel gezeigte Schwenkhebel 1h ist zweiarmig, wobei der Hebelabschnitt, der von dem Schwenkgelenk 1g, 1i in die entgegengesetzte Richtung wie der Arm, der das Eingriffsglied 1b bildet, abragt, den Nocken 1c bildet.

Die Spritze 13 wird mit der Nadelschutzkappe 14 voran über das proximale Ende des Hülsenkörpers 103 in den Hülsenkörper 103 eingeführt, wobei die Nadelschutzkappe 14 an den Eingriffsgliedern 1b vorbei bewegt wird, bis sich die Lücke zwischen dem sich verjüngenden Bereich des Spritzenkörpers und der Nadelschutzkappe 14 in Bezug auf die Längsachse auf der gleichen Position wie die Eingriffsglieder 1b befinden. Durch Verschwenken des Schwenkhebels 1h werden die Eingriffsglieder 1b in die Lücke bzw. zu der Längsachse hin geschwenkt. Die in Figur 11c gezeigte Einheit wird dann in dem Gehäuse 2 des Autoinjektors so angeordnet, dass der Halteabschnitt 2b den Schwenkhebel 1h so fixiert, dass die Eingriffsglieder 1b in dem Eingriff mit dem sich verjüngenden Abschnitt des Spritzenkörpers gehalten werden. Der Arm, an dem der Nocken 1c gebildet ist, ist in Bezug auf den Arm, an dem das Eingriffsglied 1b gebildet ist, elastisch verformbar, wodurch der Nocken 1c die in Bezug auf die Nadelschutzhülse 3 vorgesehene Aufgabe erfüllen kann. Insbesondere dient der Nocken 1a als Anschlag für die Nadelschutzhülse 3, wobei die Nadelschutzhülse 3 an dem Nocken 1a anliegt, wenn die Nadelschutzhülse in ihrer Ausgangsposition oder/und in ihrer Nadelschutzposition ist.

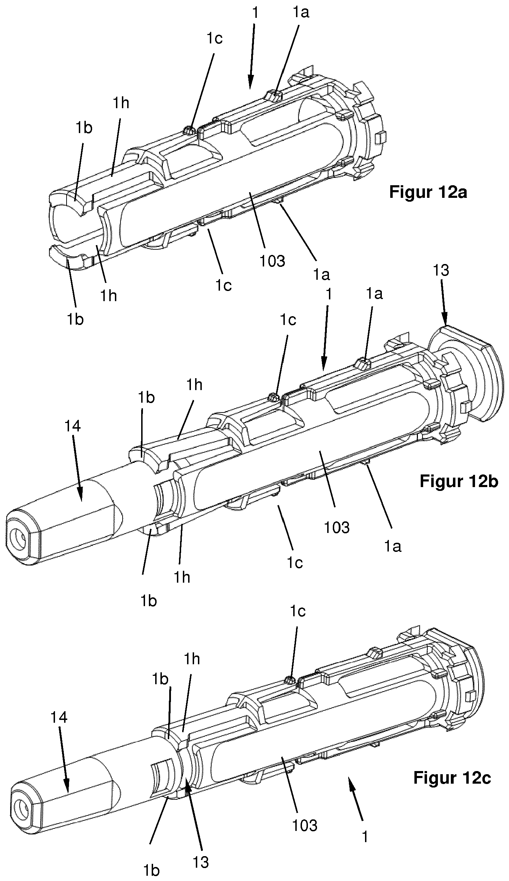

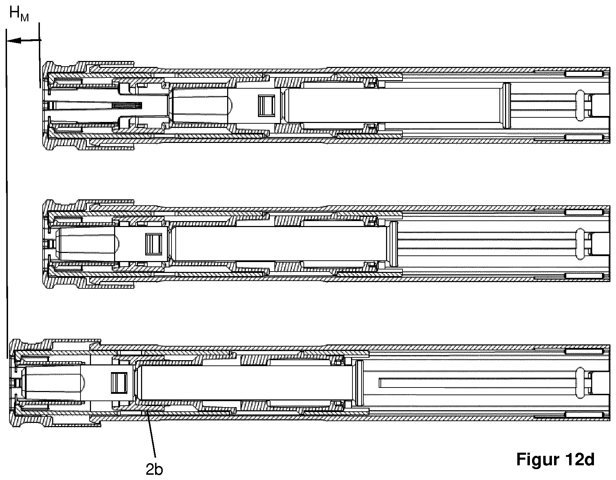

In der in den Figuren 12a-12d gezeigten fünften Ausführungsform weist das Spritzenmodul, insbesondere der Spritzenhalter 1 einen Hülsenkörper 103 auf. Der Hülsenkörper 103 weist insbesondere zwei Nocken 1c und insbesondere zwei Abragungen 1a in der hierin gezeigten Weise auf.

In dieser Variante kann das mindestens eine Eingriffsglied als Schulter 1b federnd, insbesondere an einem elastischen Arm 1h an dem Spritzenhalter gebildet sein, wobei die Spritze 13 über das proximale Ende mit der Nadel voraus in den Spritzenhalter, der vorzugsweise hülsenförmig ist, eingeschoben wird, wobei die Nadelschutzkappe 14 das mindestens eine Eingriffsglied 1b quer zur Längsachse nach außen, d. h. von der Längsachse weg auslenkt, wobei, wenn die Nadelschutzkappe 14 vollständig an dem mindestens einen Eingriffsglied 1b vorbei bewegt wurde, das mindestens eine Eingriffsglied 1b in die Lücke zwischen dem sich verjüngenden Bereich der Spritze 13 und dem proximalen Ende der Nadelschutzkappe 14 einschnappt. Die in Figur 12c gezeigte Einheit wird dann in dem Gehäuse 2 des Autoinjektors so angeordnet, dass der Halteabschnitt 2b den Arm 1h so fixiert, dass die Eingriffsglieder 1b in dem Eingriff mit dem sich verjüngenden Abschnitt des Spritzenkörpers 13 kraft- oder formschlüssig gehalten werden und aus diesem Eingriff nicht mehr herausfedern.

In den Figuren 8e, 9d, 10e, 11d, 12d sind Längsschnitte der fünf Ausführungsformen im Auslieferungszustand und für die Ausführungsformen zwei bis fünf in je nach einem Montageschritt der Spritzenmontage in den Autoinjektor in je einer Position mit teilweise und vollständig eingesetzter Spritze gezeigt. Bei vollständig eingesetzter Spritze greift ferner der zumindest eine Schnapper 4b welcher die Abziehkappe 4 aufweist in die Lücke zwischen dem Spritzenkörper 13, insbesondere dessen sich verjüngenden Bereich, und dem proximalen Ende des rigid needle shield 14 (Figur 2a, 2b).

Das zumindest eine Eingriffsglied 1b wird zusammen mit dem Spritzenhalter 1 durch einem Montagehub H_{M} welcher insbesondere als letzter Montageschritt erfolgt axial in den Bereich des Halteabschnittes 2b geschoben so dass eine kraft- oder formschlüssige Verbindung entsteht mit der verhindert wird, dass sich das mindestens eine Eingriffsglied 1b aus dem Eingriff mit dem verjüngenden Abschnitt des Spritzenkörpers 13 quer zur Längsachse, insbesondere von der Längsachse L weg oder nach aussen, bewegt. Weiter wird durch diesen Montagehub die Abziehkappe 4 in ihre distale Position bewegt, welche sie im Auslieferungszustand des Autoinjektors einnimmt, wobei die Abziehkappe 4 mittels zumindest einem Schnapphaken 4a welcher sich auf dem Spritzenhalter 1 abstützt durch den Spritzenhalter 1 bewegt wird.

**Bezugszeichenliste**

| | | | |
|---|---|---|---|
| 1 | Spritzenhalter | 8 | Sperrhülse |
| 1a | Abragung/Halteglied | 8a | Verriegelungsglied |
| 1b | Schulter/Eingriffsglied | 8b | erste Ausnehmung |
| 1c | Nocken | 8c | zweite Ausnehmung |
| 1e | Scharnierzapfen | | |
| 1f | Scharnierzapfenaufnahme | 9 | erste Feder/Ausschüttfeder 10 zweite Feder/Nadelschutzfeder |
| 1g | Scharnierzapfenaufnahme | 11 | Signalglied |
| 1i | Scharnierzapfen | 11a | erstes Eingriffsglied |
| 1h | Schwenkhebel / Arm | 11b | zweites Eingriffsglied |
| 1k | Translationsgegenanschlag | 11c | Abragung |
| 1m | Translationsanschlag | 11d | Arm |
| | | | |
| 2 | Gehäuse | 12 | Verschlusskappe |
| 2a | Ausnehmung | 12a | Rastglied |
| 2b | Ringab schnitt/Halteabschnitt | 12b | Signalanschlag |
| 2c | Betätigungsanschlag | | |
| 2e | Halteschulter | 13 | Produktbehälter/Spritze |
| | | 13a | Nadel |
| 3 | Nadelschutzhülse | 13b | Kolben |
| 3a | proximales Ende | | |
| 3b | Schnappgeometrie | 14 | rigid needle shield/Nadelschutzkappe |
| | | | |
| 4 | Abziehkappe | 15 | Schalthülse |
| 4a | Schnapphaken | 15a | Ausnehmung |
| 4b | Schnapper | | |
| | | 101 | erster Schalenkörper/Halbschale |
| 5 | Mechanikhalter | 102 | zweiter Schalenkörper/Halbschale |
| 5a | Startsignalanschlag | 103 | erster Schalenkörper/Hülsenkörper |
| 5b | Nut | 104 | zweiter Schalenkörper/ Hülsenkörper |
| 5c | Haltefederab schnitt | | |
| 5d | Vertiefung | H_{A} | Ausschütthub |
| | | H_{B} | Betätigungshub |
| 6 | Halteelement | Hs | Signalhub/erster Teilhub |
| 6a | erstes Eingriffselement | H_{K} | Startsignalhub |
| 6b | zweites Eingriffselement | H_{N} | Nadelschutzhub |
| 6c | Arm | H_{M} | Montagehub |
| 6d | Führungsstift | | |
| | | L | Längsachse |
| 7 | Vortriebsglied | | |
| 7a | erste Ausnehmung | | |
| 7b | zweite Ausnehmung | | |

## Patentansprüche

1. Autoinjektor mit einer Längsachse (L) zur Ausschüttung eines flüssigen Produkts umfassend:
a) ein Gehäuse (2) und einen in dem Gehäuse (2) angeordneten Produktbehälter (13), insbesondere Spritze, der einen verschiebbaren Kolben (13b) aufweist, wobei der Kolben (13b) zur Ausschüttung des in dem Produktbehälter (13) enthaltenen Produkts in eine Ausschüttrichtung verschiebbar ist,
b) ein Vortriebsglied (7), das während der Produktausschüttung auf den Kolben (13b) wirkt, und eine erste Feder (9), die auf das Vortriebsglied (7) wirkt, wobei die erste Feder (9) so stark vorgespannt ist, dass sie das Produkt aus dem Produktbehälter (13) durch Verschieben des Vortriebsglieds (7) um einen Ausschütthub (H_{A}) und des Kolbens (13b) ausschütten kann,
**gekennzeichnet durch**
c) ein Signalglied (11), einen Signalanschlag (12b) und eine zweite Feder (10), welche auf das Signalglied (11) eine entgegen der Ausschüttrichtung wirkende Federkraft ausübt, wobei das Signalglied (11) während einem ersten Teilhub (Hs) des Ausschütthubs (H_{A}) über ein erstes Eingriffsglied (11a) mit dem Vortriebsglied (7) axialfest gekoppelt ist, so dass das Signalglied (11) während des Verschiebens des Vortriebsglieds (7) in die Ausschüttrichtung mitgenommen und die zweite Feder (10) gespannt wird, und wobei bei gelöster axialfester Kopplung zwischen Signalglied (11) und Vortriebsglied (7) das Signalglied (11) mittels der zweiten Feder (10) entgegen der Ausschüttrichtung und relativ zu dem Vortriebsglied (7) oder/und dem Gehäuse (2) beschleunigbar ist, wodurch das von der zweiten Feder (10) beschleunigte Signalglied (11) an dem Signalanschlag (12b) anschlägt und ein akustisches und/oder taktiles Signal erzeugt wird.

2. Autoinjektor nach Anspruch 1, **dadurch gekennzeichnet, dass** das erste Eingriffsglied (11a) in das Vortriebsglied (7) lösbar eingreift, wodurch das Vortriebsglied (7) axialfest mit dem Signalglied (11) gekoppelt ist, wobei die axialfeste Kopplung zwischen dem Vortriebsglied (7) und dem Signalglied (11) gelöst ist, wenn das erste Eingriffsglied (11a) aus dem Eingriff mit dem Vortriebsglied (7) ausgerückt oder herausgedrückt ist.

3. Autoinjektor nach einem der vorhergehenden Ansprüche, **dadurch gekennzeichnet, dass** der Signalanschlag (12b) von dem Gehäuse (2) oder einem zumindest axialfest mit dem Gehäuse (2) verbundenen Element (12) gebildet ist, und dass der Signalanschlag (12b) entlang der Längsachse (L) des Autoinjektors in einer Flucht mit dem Signalglied (11) angeordnet ist.

4. Autoinjektor nach einem der vorhergehenden Ansprüche, **dadurch gekennzeichnet, dass** der Produktbehälter (13) entlang der Längsachse (L) des Autoinjektors unverschiebbar in dem Gehäuse aufgenommen ist.

5. Autoinjektor nach einem der vorhergehenden Ansprüche, **gekennzeichnet durch** eine auf die zweite Feder (10) wirkende Nadelschutzhülse (3), welche für die Auslösung der Produktausschüttung aus ihrer Ausgangsposition relativ zu dem Gehäuse (2) und entlang der Längsachse (L) des Autoinjektors in eine proximale Richtung um einen Betätigungshub (H_{B}) verschiebbar ist, wodurch die zweite Feder (10) gespannt und die Produktausschüttung ausgelöst wird.

6. Autoinjektor nach Anspruch 5, **gekennzeichnet durch** ein Schaltmodul (8, 15), welches kinematisch oder/und geometrisch zwischen der zweiten Feder (10) und der Nadelschutzhülse (3) angeordnet ist, wobei das Schaltmodul (8, 15) von der Nadelschutzhülse (3) in die proximale Richtung mitgenommen wird, wenn die Nadelschutzhülse (3) aus ihrer Ausgangsposition in die proximale Richtung verschoben wird.

7. Autoinjektor nach Anspruch 6, **dadurch gekennzeichnet, dass** das Signalglied (11) ein zweites Eingriffsglied (11b) aufweist, welches durch die Ausrückbewegung des ersten Eingriffsglieds (11a) aus dem Vortriebsglied (7) in einen axialfesten Eingriff mit der Nadelschutzhülse (3) oder dem Schaltmodul (8, 15) bewegbar ist, wobei das erste Eingriffsglied (11a) und das zweite Eingriffsglied (11b) so aufeinander abgestimmt sind, dass das zweite Eingriffsglied (11b) bereits axialfest in die Nadelschutzhülse (3) oder das Schaltmodul (8, 15) eingreift, wenn das erste Eingriffsglied (11a) noch nicht vollständig aus dem Eingriff mit dem Vortriebsglied (7) gelöst ist.

8. Autoinjektor nach Anspruch 7, **dadurch gekennzeichnet, dass** das Vortriebsglied (7) mittels der ersten Feder (9) relativ zu dem Signalglied (11) in die distale Richtung bewegbar ist, wenn das erste Eingriffsglied (11a) aus dem Eingriff mit dem Vortriebsglied (7) und das zweite Eingriffsglied (11b) in dem Eingriff mit der Nadelschutzhülse (3) oder dem Schaltmodul (8, 15) ist.

9. Autoinjektor nach Anspruch 7 oder 8, **dadurch gekennzeichnet, dass** das Vortriebsglied (7) das zweite Eingriffsglied (11) daran hindert, aus dem axialfesten Eingriff mit der Nadelschutzhülse (3) oder dem Schaltmodul (8, 15) auszurücken, wenn sich das Vortriebsglied (7) relativ zu dem Signalglied (11) in distale Richtung bewegt, wobei das Vortriebsglied (7) am Ende des Ausschütthubs (H_{A}) dem zweiten Eingriffsglied (11b) erlaubt, aus dem Eingriff mit der Nadelschutzhülse (3) oder dem Schaltmodul (8, 15) auszurücken, wodurch das Signalglied (11) von der zweiten Feder (10) entgegen die Ausschüttrichtung beschleunigt wird und an dem Signalanschlag (12b) anschlägt.

10. Autoinjektor nach einem der vorhergehenden Ansprüche, **gekennzeichnet durch** ein Halteelement (6), an dem sich ein Ende der ersten Feder (9) abstützt und das ein erstes Eingriffselement (6a) aufweist, wobei das erste Eingriffselement (6a) vor dem Auslösen der Produktausschüttung in das Vortriebsglied (7) eingreift, wodurch das Vortriebsglied (7) daran gehindert wird, sich relativ zu dem Halteelement (6) in Ausschüttrichtung zu bewegen, wobei dieser Eingriff des ersten Eingriffselements (6a) für die Produktausschüttung lösbar ist, so dass die erste Feder (9) das Vortriebsglied (7) relativ zu dem Halteelement (6) in die Ausschüttrichtung verschieben kann.

11. Autoinjektor nach den Ansprüchen 10 und 6, **dadurch gekennzeichnet, dass** das Halteelement (6) ein zweites Eingriffselement (6b) aufweist, welches durch die Ausrückbewegung des ersten Eingriffselements (6a) aus dem Vortriebsglied (7) in einen axialfesten Eingriff mit der Nadelschutzhülse (3) oder dem Schaltmodul (8, 15) bewegbar ist, wobei das erste Eingriffselement (6a) und das zweite Eingriffselement (6b) so aufeinander abgestimmt sind, dass das zweite Eingriffselement (6b) bereits axialfest in die Nadelschutzhülse (3) oder das Schaltmodul (8, 15) eingreift, wenn das erste Eingriffselement (6a) noch nicht vollständig aus dem Eingriff mit dem Vortriebsglied (7) gelöst ist.

12. Autoinjektor nach Anspruch 11, **dadurch gekennzeichnet, dass** das Vortriebsglied (7) das zweite Eingriffselement (6b) daran hindert, aus dem axialfesten Eingriff mit der Nadelschutzhülse (3) oder dem Schaltmodul (8, 15) auszurücken, wenn sich das Vortriebsglied (7) relativ zu dem Signalglied (11) in distale Richtung bewegt und am Ende des Ausschütthubs (H_{A}) stehenbleibt.

13. Autoinjektor nach Anspruch 12, **dadurch gekennzeichnet, dass** das Schaltmodul (8, 15) eine Schalthülse (15) und Sperrhülse (8) aufweist, wobei die Sperrhülse (8) ein unidirektional wirkendes Verriegelungsglied (8a) aufweist, welches in die Schalthülse (15) eingreift, wobei die Schalthülse (15) bei ihrer Bewegung relativ zu dem Gehäuse (2) in die proximale Richtung die Sperrhülse (8) über das Verriegelungsglied (8a) mitnimmt und bei ihrer Bewegung relativ zu dem Gehäuse (2) in proximale Richtung relativ zu der Sperrhülse (8) in eine weitere Sperrposition verschoben wird, in der das Verriegelungsglied (8a) eine Bewegung der Schalthülse (15) relativ zu der Sperrhülse (8) in die proximale Richtung sperrt.

14. Autoinjektor nach einem der Ansprüche 5 bis 13, **dadurch gekennzeichnet, dass** die Nadelschutzhülse (3) nach der Produktausschüttung, wenn das Vortriebsglied (7) um den Ausschütthub (H_{A}) in die distale Richtung verschoben ist, von der zweiten Feder (10) relativ zu dem Gehäuse (2) in die distale Richtung, insbesondere um einen Nadelschutzhub (H_{N}) in eine Nadelschutzposition verschiebbar ist, in der die Nadelschutzhülse (3) distal über die Nadelspitze einer Injektionsnadel (13a) des Produktbehälters (13) steht.

15. Autoinjektor nach Anspruch 14, **gekennzeichnet durch** ein Verriegelungsglied (8a), welches die Nadelschutzhülse (3) in ihrer Nadelschutzposition relativ zu dem Gehäuse (2) gegen Zurückschieben in die proximale Richtung zumindest so verriegelt, dass die Nadelspitze nicht aus dem distalen Ende der Nadelschutzhülse (3) hervortreten kann.

## Claims

1. An autoinjector with a longitudinal axis (L) for dispensing a liquid product, comprising:
a) a housing (2) and a product container (13), in particular a syringe, arranged in the housing, the product container comprising a piston (13b) displaceable in a dispensing direction for dispensing the product contained in the product container;
b) a drive member (7) for displacing the piston during the product dispensing; and a first spring (9) for displacing the drive member, wherein the first spring is pretensioned such that it can dispense the product from the product container by displacing the drive member by a dispensing stroke (H_{A}) and by displacing the piston;
**characterized by**
c) a signal element (11); a signal stop (12b); and a second spring (10), wherein the second spring exerts a spring force on the signal element opposite the dispensing direction, and wherein the signal element, during a first part stroke (Hs) of the dispensing stroke (H_{A}) and via a first engagement element (11a), is coupled with the drive member in an axial coupling such that, as the drive member is displaced, the signal element is displaced in the dispensing direction and the second spring is tensed, and wherein when the axial coupling between the signal element and the drive member is released, the signal element may be accelerated by the second spring opposite the dispensing direction and relative to one or more of the drive member or the housing, whereby the signal element accelerated by the second spring strikes the signal stop of the housing thereby generating one or more of an acoustic or a tactile signal.

2. The autoinjector according to claim 1, wherein the first engagement element detachably engages the drive member to axially couple the drive member and the signal element, wherein the axial coupling between the drive member and the signal element is released when the first engagement element is disengaged or pushed out of engagement with the drive member.

3. The autoinjector according to one of the preceding claims, wherein the signal stop is formed by the housing or by an element (12) axially fixedly connected to the housing, and wherein the signal stop is arranged along the longitudinal axis of the housing and in alignment with the signal element.

4. The autoinjector according to one of the preceding claims, wherein the product container is arranged non-displaceable along the longitudinal axis in the housing.

5. The autoinjector according to one of the preceding claims, further comprising a needle protective sleeve (3) for acting on the second spring and for triggering the product dispensing, wherein the needle protective sleeve is moveable from its starting position relative to the housing and along the longitudinal axis of the autoinjector in the proximal direction by an activation stroke (H_{B}), whereby the second spring is tensioned and dispensing of the product is triggered.

6. The autoinjector according to claim 5, further comprising a switch module (8, 15) arranged kinematically and/or geometrically between the second spring and the needle protective sleeve, wherein the switch module is transported in the proximal direction by the needle protective sleeve when the needle protective sleeve is displaced from its starting position in the proximal direction.

7. The autoinjector according to claim 6, wherein the signal element comprises a second engagement element (11b), which can be moved into an axially fixed engagement with the needle protective sleeve or the switch module by a decoupling movement of the axial coupling between the drive member and the first engagement element, wherein the first engagement element and the second engagement element are coordinated with one another such that the second engagement element engages in the axially fixed engagement with the needle protective sleeve or the switch module when the first engagement element has not yet detached from the engagement with the drive member.

8. The autoinjector according to claim 7, wherein the drive member is moveable in the distal direction relative to the signal element by means of the first spring when the first engagement element is detached from the drive member, and when the second engagement element is in engagement with the needle protective sleeve or the switch module.

9. The autoinjector according to claim 7 or claim 8, wherein the drive member prevents the second engagement element from moving out of the axially fixed engagement with the needle protective sleeve or with the switch module when the drive member moves in the distal direction relative to the signal element, wherein at the end of the dispensing stroke, the drive member allows the second engagement element to move out of engagement with the needle protective sleeve or with the switch module, whereby the signal element is accelerated by the second spring against the dispensing direction and strikes the signal stop.

10. The autoinjector according to one of the preceding claims, further comprising a holding element (6) on which one end of the first spring is supported and which comprises a first engagement element (6a), wherein the first engagement element engages the drive member before product dispensing is triggered, whereby the drive member is prevented from moving in the dispensing direction relative to the holding element, wherein said engagement of the first engagement element can be released for the product dispensing so that the first spring can move the drive member in the dispensing direction relative to the holding element.

11. The autoinjector according to claim 10 and claim 6, wherein the holding element comprises a second engagement element (6b), which can be moved by a disengaging movement of the first engagement element of the holding element out of the drive member into an axially fixed engagement with the needle protective sleeve or the switch module, wherein the first engagement element of the holding element and the second engagement element of the holding element are coordinated with one another such that the second engagement element of the holding element engages the needle protective sleeve or the switch module in an axially fixed manner when the first engagement element of the holding element has not yet decoupled from its engagement in the drive member.

12. The autoinjector according to claim 11, wherein the drive member prevents the second engagement element of the holding element from moving out of the axially fixed engagement with the needle protective sleeve or with the switch module when the drive member moves in the distal direction relative to the signal element and stops at the end of the dispensing stroke.

13. The autoinjector according to claim 12, wherein the switch module comprises a switch sleeve (15) and a blocking sleeve (8), wherein the blocking sleeve comprises a unidirectionally acting locking element (8a) which engages the switch sleeve, wherein during its movement relative to the housing in the proximal direction, the switch sleeve transports the blocking sleeve via the locking element, and during its movement relative to the housing in proximal direction, the blocking sleeve is moved into a further blocking position in which the locking element blocks a movement of the switch sleeve relative to the blocking sleeve in the proximal direction.

14. The autoinjector according to one of claims 5 to 13, wherein upon the drive member being displaced by the dispensing stroke in the distal direction, the needle protective sleeve can be moved by the second spring in the distal direction relative to the housing by a needle protective stroke (H_{N}) into the needle protective position in which the needle protective sleeve protrudes distally beyond the needle tip of an injection needle (13a) of the product container.

15. The autoinjector according to claim 14, further comprising a locking element (8a) which blocks the needle protective sleeve in its needle protective position relative to the housing against being pushed back in the proximal direction such that the needle tip cannot protrude from a distal end of the needle protective sleeve.

## Revendications

1. Auto-injecteur avec un axe longitudinal (L) pour la distribution d'un produit liquide comprenant :
a) un boîtier (2) et un récipient de produit (13) agencé dans le boîtier (2), en particulier une seringue qui présente un piston coulissant (13b), le piston (13b) étant à apte à coulisser pour la distribution du produit contenu dans le récipient de produit (13) dans un sens de distribution,
b) un organe d'avancement (7) qui agit sur le piston (13b) pendant la distribution de produit, et un premier ressort (9) qui agit sur l'organe d'avancement (7), le premier ressort (8) étant précontraint si fortement qu'il peut distribuer le produit du récipient de produit (13) par coulissement de l'organe d'avancement (7) d'une course de distribution (H_{A}) et du piston (13b),
**caractérisé par**
c) un organe de signal (11), une butée de signal (12b) et un second ressort (10) qui exerce sur l'organe de signal (11) une force de ressort agissant dans le sens inverse au sens de distribution, dans lequel l'organe de signal (11) est accouplé de manière axialement fixe pendant une première course partielle (Hs) de la course de distribution (H_{A}) par le biais d'un premier organe de mise en prise (11a) à l'organe d'avancement (7) de sorte que l'organe de signal (11) soit entraîné pendant le coulissement de l'organe d'avancement (7) dans le sens de distribution et le second ressort (10) soit tendu, et dans lequel en cas d'accouplement axialement fixe détaché entre l'organe de signal (11) et l'organe d'avance (7) l'organe de signal (11) peut être accéléré au moyen du second ressort (10) dans le sens inverse au sens de distribution et par rapport à l'organe d'avancement (7) et/ou au boîtier (2), moyennant quoi l'organe de signal (11) accéléré par le second ressort (10) bute contre la butée de signal (12b) et un signal acoustique et/ou tactile est généré.

2. Auto-injecteur selon la revendication 1, **caractérisé en ce que** le premier organe de mise en prise (11a) se met en prise de manière détachable dans l'organe d'avancement (7), moyennant quoi l'organe d'avancement (7) est accouplé de manière axialement fixe à l'organe de signal (11), dans lequel l'accouplement axialement fixe est détaché entre l'organe d'avancement (7) et l'organe de signal (11), lorsque le premier organe de mise en prise (11a) est désengagé ou expulsé de la mise en prise avec l'organe d'avancement (7).

3. Auto-injecteur selon l'une des revendications précédentes, **caractérisé en ce que** la butée de signal (12b) est formée par le boîtier (2) ou un élément (12) raccordé au moins de manière axialement fixe au boîtier (2), et que la butée de signal (12b) est agencée le long de l'axe longitudinal (L) de l'auto-injecteur dans un alignement avec l'organe de signal (11).

4. Auto-injecteur selon l'une des revendications précédentes, **caractérisé en ce que** le récipient de produit (13) est reçu le long de l'axe longitudinal (L) de l'auto-injecteur sans pouvoir coulisser dans le boîtier.

5. Auto-injecteur selon l'une des revendications précédentes, **caractérisé par** une douille de protection d'aiguille (3) agissant sur le second ressort (10) qui peut coulisser pour le déclenchement de la distribution de produit de sa position de départ par rapport au boîtier (2) et le long de l'axe longitudinal (L) de l'auto-injecteur dans un sens proximal d'une course d'actionnement (H_{B}), moyennant quoi le second ressort (10) est tendu et la distribution de produit est déclenchée.

6. Auto-injecteur selon la revendication 5, **caractérisé par** un module de commutation (8, 15) qui est agencé de manière cinématique et/ou géométrique entre le second ressort (10) et la douille de protection d'aiguille (3), dans lequel le module de commutation (8, 15) est entraîné par la douille de protection d'aiguille (3) dans le sens proximal lorsque la douille de protection d'aiguille (3) est coulissée de sa position de départ dans le sens proximal.

7. Auto-injecteur selon la revendication 6, **caractérisé en ce que** l'organe de signal (11) présente un second organe de mise en prise (11b) qui est mobile par le mouvement de désengagement du premier organe de mise en prise (11a) hors de l'organe d'avancement (7) dans une mise en prise axialement fixe avec la douille de protection d'aiguille (3) ou le module de commutation (8, 15), dans lequel le premier organe de mise en prise (11a) et le second organe de mise en prise (11b) sont adaptés l'un à l'autre de sorte que le second organe de mise en prise (11b) se mette déjà en prise de manière axialement fixe dans la douille de protection d'aiguille (3) ou le module de commutation (8, 15) lorsque le premier organe de mise en prise (11a) n'est pas encore complètement détaché de la mise en prise avec l'organe de d'avancement (7).

8. Auto-injecteur selon la revendication 7, **caractérisé en ce que** l'organe d'avancement (7) est mobile au moyen du premier ressort (9) par rapport à l'organe de signal (11) dans le sens distal lorsque le premier organe de mise en prise (11a) est hors de la mise en prise avec l'organe d'avancement (7) et le second organe de mise en prise (11b) est dans la mise en prise avec la douille de protection d'aiguille (3) ou le module de commutation (8, 15).

9. Auto-injecteur selon la revendication 7 ou 8, **caractérisé en ce que** l'organe d'avancement (7) empêche le second organe de mise en prise (11b) de se désengager de la mise en prise axialement fixe avec la douille de protection d'aiguille (3) ou le module de commutation (8, 15) lorsque l'organe d'avancement (7) se déplace par rapport à l'organe de signal (11) dans le sens distal, dans lequel l'organe d'avancement (7) permet à la fin de la course de distribution (H_{A}) au second organe de mise en prise (11b) de se désengager de la mise en prise avec la douille de protection d'aiguille (3) ou le module de commutation (8, 15), moyennant quoi l'organe de signal (11) est accéléré par le second ressort (10) dans le sens inverse au sens de distribution et bute contre la butée de signal (12b).

10. Auto-injecteur selon l'une des revendications précédentes, **caractérisé par** un élément de retenue (6) contre lequel une extrémité du premier ressort (9) s'appuie et qui présente un premier élément de mise en prise (6a), dans lequel le premier élément de mise en prise (6a) se met en prise avant le déclenchement de la distribution de produit dans l'organe de d'avancement (7), moyennant quoi l'organe d'avancement (7) est empêché de se déplacer par rapport à l'élément de retenue (6) dans le sens de distribution, dans lequel cette mise en prise du premier élément de mise en prise (6a) pour la distribution de produit est détachable de sorte que le premier ressort (9) puisse faire coulisser l'organe d'avancement (7) par rapport à l'élément de retenue (6) dans le sens de distribution.

11. Auto-injecteur selon les revendications 10 et 6, **caractérisé en ce que** l'élément de retenue (6) présente un second élément de mise en prise (6b) qui peut être déplacé par le mouvement de désengagement du premier élément de mise en prise (6a) de l'organe d'avancement (7) dans une mise en prise axialement fixe avec la douille de protection d'aiguille (3) ou le module de commutation (8, 15), dans lequel le premier élément de mise en prise (6a) et le second élément de mise en prise (6b) sont adaptés l'un à l'autre de sorte que le second élément de mise en prise (6b) se mette déjà en prise de manière axialement fixe dans la douille de protection d'aiguille (3) ou le module de commutation (8, 15) lorsque le premier élément de mise en prise (6a) n'est pas encore complètement détaché de la mise en prise avec l'organe d'avancement (7).

12. Auto-injecteur selon la revendication 11, **caractérisé en ce que** l'organe de d'avancement (7) empêche le second élément de mise en prise (6b) de se désengager de la mise en prise axialement fixe avec la douille de protection d'aiguille (3) ou le module de commutation (8, 15) lorsque l'organe d'avancement (7) se déplace par rapport à l'organe de signal (11) dans le sens distal et reste immobile à la fin de la course de distribution (H_{A}).

13. Auto-injecteur selon la revendication 12, **caractérisé en ce que** le module de commutation (8, 15) présente une douille de commutation (15) et une douille de blocage (8), dans lequel la douille de blocage (8) présente un organe de verrouillage (8a) agissant de manière unidirectionnelle qui se met en prise dans la douille de commutation (15), dans lequel la douille de commutation (15) entraîne lors de son mouvement par rapport au boîtier (2) dans le sens proximal la douille de blocage (8) par le biais de l'organe de verrouillage (8a) et est coulissée lors de son mouvement par rapport au boîtier (2) dans le sens proximal par rapport à la douille de blocage (8) dans une autre position de blocage, dans laquelle l'organe de verrouillage (8a) bloque un mouvement de la douille de commutation (15) par rapport à la douille de blocage (8) dans le sens proximal.

14. Auto-injecteur selon l'une des revendications 5 à 13, **caractérisé en ce que** la douille de protection d'aiguille (3) peut coulisser après la distribution de produit lorsque l'organe de d'avancement (7) est coulissé de la course de distribution (H_{A}) dans le sens distal, du second ressort (10) par rapport au boîtier (2) dans le sens distal, en particulier d'une course de protection d'aiguille (H_{N}) dans une position de protection d'aiguille, dans laquelle la douille de protection d'aiguille (3) est distale au-dessus de la pointe d'aiguille d'une aiguille d'injection (13a) du récipient de produit (13).

15. Auto-injecteur selon la revendication 14, **caractérisé par** un organe de verrouillage (8a), qui verrouille la douille de protection d'aiguille (3) dans sa position de protection d'aiguille par rapport au boîtier (2) contre le repoussement dans le sens proximal au moins de sorte que la pointe d'aiguille ne puisse pas sortir de l'extrémité distale de la douille de protection d'aiguille (3).
